# EUROPEAN PATENT SPECIFICATION

(11) **EP 2 883 491 B1**
(45) Date of publication and mention of the grant of the patent: **19.04.2017**
(21) Application number: 14196626.7
(22) Date of filing: 05.12.2014
(51) Int. Cl.: A61B 1/005

(54) **Endoscope**
Endoskop
Endoscope

(30) Priority: 12.12.2013 JP 2013257048; 12.12.2013 JP 2013256681; 28.02.2014 JP 2014038489
(43) Date of publication of application: 17.06.2015
(73) Proprietor: Panasonic Intellectual Property Management Co., Ltd., Osaka-shi, Osaka 540-6207 (JP)
(72) Inventor: Kohno, Haruhiko, Osaka-shi, Osaka, 540-6207 (JP)
(74) Representative: Eisenführ Speiser

(56) References cited:
- WO-A1-2013/108671
- US-A- 4 203 430
- US-A1- 2007 282 371

## Description

### BACKGROUND OF THE INVENTION

### 1. Field of the Invention

The present invention relates to an endoscope for imaging the inside of an observation object which may not be directly observed from the outside.

### 2. Description of the Related Art

Conventionally, in medical and industrial fields, an endoscope for imaging a patient's body and the insides of devices and structures has come into wide use. As this type of endoscope, a configuration is known in which, in an insertion section inserted into an observation object, an image is formed on a light receiving surface of an imaging device by an objective lens system receiving light from an imaging portion and the focused light is converted into electric signals to be transmitted as video signals to an external image processing device or the like through signal cables.

A plurality of parts such as an imaging device and a lens to form a light image on an imaging surface of the imaging device are disposed in a rigid section provided at a tip end of this type of endoscope. For example, the endoscope has a structure in which the parts are placed in a housing by integrally holding optical elements such as a plurality of lenses in a lens tube and supporting the lens tube and the imaging device by a holder. In recent years, a configuration is known in which an imaging direction, namely, a visual field is changed based on the operation of an operator or the like by connecting the rigid section to the bendable insertion section.

As such an endoscope, there is disclosed, for example, an image endoscope including: a shaft which has a proximal end and a distal end and has one or more holes therein; one or more light emitting diodes (LEDs) which are disposed in the distal end of the shaft or disposed adjacent to the distal end for illuminating tissue; an image assembly which is disposed at the distal end of the shaft and includes an image sensor for generating an image of the tissue; a plurality of control cables which are selectively tensioned so as to bend the shaft in a desired direction; a deformable articulated joint which includes a plurality of links bonded together with a spring segment, the plurality of links being bendable under tension of one or more control cables of the plurality of control cables, at least a portion of the spring segment being disposed within a concave section defined on an inside surface of each of the plurality of the links; and an external sheath on the articulated joint (see Related Art 1). According to Related Art 1, an image can be captured in any direction by bending the articulated joint and a wider range of the image can be observed.

Related Art 1: Japanese Patent No. 4676427 US 2007/0282371 A1 discloses a surgical instrument, according to the preamble of claim 1, , which includes a distal tool, a rigid or flexible elongated shaft that supports the distal tool, and a proximal handle or control member, where the tool and the handle are coupled to the respective distal and proximal ends of the elongated shaft via distal and proximal bendable motion members. Actuation means extends between said distal and proximal members whereby any deflection of said control handle with respect to said elongated instrument shaft causes a corresponding bending of said distal motion member for control of said working member. The proximal movable member comprises a movable ring assembly supported from the handle and adapted for three dimensional motion relative to the handle. A manually rotatable member may be arranged adjacent to the control handle for manually rotating the instrument shaft and working member relative to the control handle. A locking member may be supported from the control handle. The locking member is manually operable by a user and includes ä follower the position of which is responsive to the position of the movable members.

In surgery, surgical instruments such as a forceps and an ultrasonic scalpel in addition to an endoscope are inserted into a body cavity. However, due to a positional relationship between the endoscope and the other instruments, for example, a movement direction of the ultrasonic scalpel does not sometimes coincide with a direction (top and bottom or left and right) of an image captured by the endoscope. The resolution of the "direction discordance" is required in order to perform the surgery with more safety. The "direction discordance" is resolved by rotating the captured image at any angle while the imaging direction is maintained.

However, in the technology disclosed in Related Art 1, a visual field is movable by bending the insertion section configured of the articulated joint in any direction, but the image is not rotatable while the imaging direction is maintained in a state in which the insertion section is bent. Moreover, when the insertion section is linear, the image is rotated when an endoscope body is rotated about a direction in which the insertion section extends. However, when the insertion section is bent, the visual field is significantly moved along with the rotation of the endoscope body.

### SUMMARY OF THE INVENTION

According to an aspect of the present invention, an endoscope includes an insertion section as defined in claim 1.

### BRIEF DESCRIPTION OF DRAWINGS

The present invention is further described in the detailed description which follows, in reference to the noted plurality of drawings by way of non-limiting examples of exemplary embodiments of the present invention, in which like reference numerals represent similar parts throughout the several views of the drawings, and wherein:
FIG. 1 is a view illustrating an overall configuration of an endoscope according to a first exemplary embodiment of the present invention;
FIG. 2 is a perspective view illustrating a configuration of an insertion section;
FIG. 3 is a view for illustrating a basic configuration of a connection section and a relationship between a state of the connection section and a bent state of the insertion section;
FIG. 4 is a view for illustrating the basic configuration of the connection section and the relationship between the state of the connection section and the bent state of the insertion section;
FIG. 5 is a view illustrating schematic configurations of a traction member and a wire guide constituting the connection section when viewed from the front;
FIG. 6A is a view for illustrating a first modification example of the connection section;
FIG. 6B is a view for illustrating the first modification example of the connection section;
FIG. 7A is a perspective view illustrating a configuration of a rigid section attached to an idle end of the insertion section;
FIG. 7B is a perspective view illustrating the configuration of the rigid section attached to the idle end of the insertion section;
FIG. 8 is a view for illustrating a configuration of transferring driving force for a tilt operation to the rigid section;
FIG. 9 is a view for illustrating the configuration of transferring the driving force for the tilt operation to the rigid section;
FIG. 10A is a view for illustrating a second modification example of the connection section;
FIG. 10B is a view for illustrating a third modification example of the connection section;
FIG. 11A is an exploded perspective view illustrating a specific configuration of the connection section;
FIG. 11B is a view for illustrating a configuration of enlarging a traction amount of the connection section;
FIG. 11C is a view for illustrating a principal part related to the traction amount of the connection section;
FIG. 12 is a view for illustrating a fourth modification example of the connection section;
FIG. 13 is a view for illustrating a bent state of the insertion section in the fourth modification example;
FIG. 14 is a view for illustrating a fifth modification example of the connection section;
FIG. 15 is a view for illustrating a schematic configuration of the endoscope and a bent state of the insertion section in the fifth modification example;
FIG. 16 is a perspective view illustrating a configuration of a first connection section which connects an insertion section to a rigid section in an endoscope according to a second exemplary embodiment of the present invention;
FIG. 17A is a view schematically illustrating the rigid section and a state in which a transmission cable is not mounted to the insertion section;
FIG. 17B is a view schematically illustrating the rigid section and a state in which the transmission cable is mounted to the insertion section;
FIG. 18A is a view schematically illustrating a cross section taken along XVIIIa - XVIIIa in FIG. 17B;
FIG. 18B is a view schematically illustrating a cross section taken along XVIIIb - XVIIIb in FIG. 17B;
FIG. 19A is a perspective view illustrating a configuration of a second connection section which connects a linear section to an insertion section in an endoscope according to a third exemplary embodiment of the present invention;
FIG. 19B is a perspective view illustrating the configuration of the second connection section which connects the linear section to the insertion section in the endoscope according to the third exemplary embodiment of the present invention;
FIG. 19C is a perspective view illustrating the configuration of the second connection section which connects the linear section to the insertion section in the endoscope according to the third exemplary embodiment of the present invention;
FIG. 20 is a perspective view illustrating a traction coupling connection section of a control wire in the second connection section;
FIG. 21A is a view schematically illustrating a rigid section, the insertion section, and a state in which a transmission cable is not mounted to the linear section;
FIG. 21B is a view schematically illustrating the rigid section, the insertion section, and a state in which the transmission cable is mounted to the linear section;
FIG. 22A is a view schematically illustrating a cross section taken along XXIIa - XXIIa in FIG. 21B; and
FIG. 22B is a view schematically illustrating a cross section taken along XXIIb - XXIIb in FIG. 21B.

### DETAILED DESCRIPTION OF THE PREFERRED EMBODIMENTS

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show structural details of the present invention in more detail than is necessary for the fundamental understanding of the present invention, the description taken with the drawings making apparent to those skilled in the art how the forms of the present invention may be embodied in practice.

### [FIRST EXEMPLARY EMBODIMENT]

Hereinafter, a first exemplary embodiment of the present invention will be described with reference to the drawings. In principle, directions used in the description comply with the directions illustrated in each drawing. However, the direction in which a member extends in the member formed in a cylindrical shape or a bar shape, or the direction of a rotary shaft in a rotating member is also referred to as "an axial direction". In addition, the direction directed inward and outward about an axis is also referred to as "a radial direction" and the direction of rotating about the axis is also referred to as "a circumferential direction". In addition, in a member having a rectangular cross-section perpendicular to the axial direction, the direction is also referred to as "a radial direction" or "a circumferential direction" for convenience.

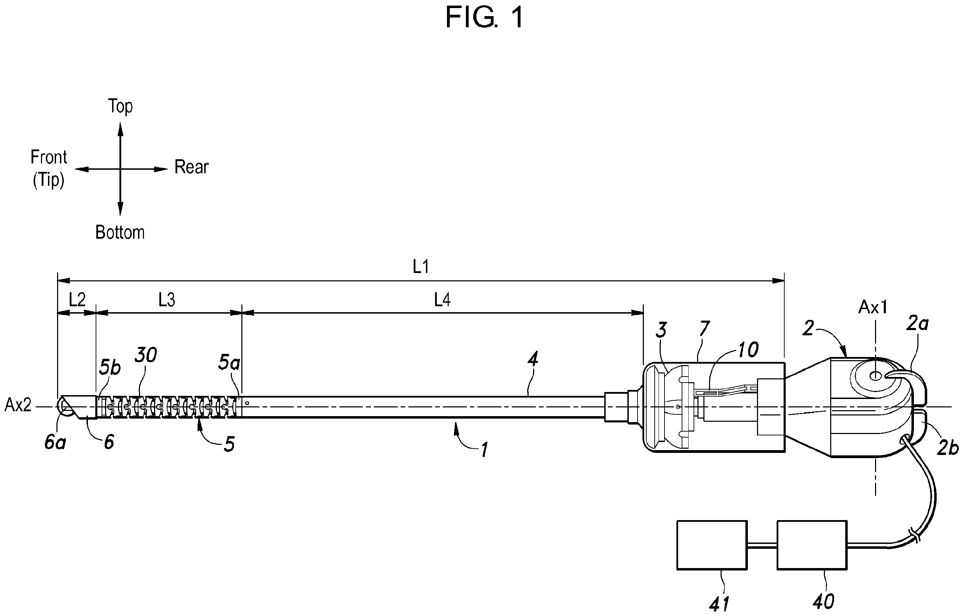

FIG. 1 is a view illustrating an overall configuration of endoscope 1 according to a first exemplary embodiment of the present invention. As shown in FIG. 1, endoscope 1 mainly includes grip section 2, connection section 3, linear section 4 which is not bendable and is of a linear pipe shape connected to grip section 2 through connection section 3, insertion section 5 configured to be bendable, rigid section 6 in which imaging unit 6a as an example of a functional member is stored, and rotation operation section 7 which rotates about an extension direction of linear section 4.

Here, length L1 from a tip end of rigid section 6 to a rear end of rotation operation section 7 is about 600 mm, rigid section 6 has length L2 of about 15 mm, insertion section 5 has length L3 of about 60 mm, linear section 4 has length L4 of about 450 mm, and each of rigid section 6, insertion section 5, and linear section 4 has an outer diameter of about 10 mm at a maximum portion thereof. When surgery is performed, rigid section 6 and insertion section 5 from among them are guided to an affected area through a trocar and a trocar tube. Meanwhile, the surgery procedure is performed in a state in which a portion of linear section 4 is exposed outside a body.

Grip section 2 is provided with first operation portion 2a to operate insertion section 5 so as to bend the same and second operation portion 2b to operate an imaging direction by imaging unit 6a mounted on rigid section 6. When an operator or the like operates first operation portion 2a, insertion section 5 is bent in a predetermined direction (for instance, in a downward direction) according to an operation amount thereof and the imaging direction of imaging unit 6a provided on rigid section 6 is changed, namely, a visual field is moved. First operation portion 2a in grip section 2 is rotatable about first axis Ax1, and the rotation direction coincides with a bending direction of insertion section 5 in consideration with operability.

In the following description, an operation in which insertion section 5 is bent by the operation of first operation portion 2a and thus the visual field is moved is also referred to as "a bending operation", an angle formed by a direction in which the tip end of rigid section 6 is directed by bending and an axial direction (second axis Ax2) of linear section 4 is also referred to as "a bending angle", and a direction in which the tip end of rigid section 6 is directed by bending in the front view is also referred to as "a bending direction". For example, a case in which insertion section 5 is bent such that the tip end of rigid section 6 is directed downward (upward) is expressed as "it being bent downward (upward)".

In addition, second operation portion 2b is also rotated about first axis Ax1. When the operator or the like operates second operation portion 2b, the visual field of imaging unit 6a pivoted on rigid section 6 is moved between the forward direction and the downward direction herein. In the following description, an operation in which the visual field is moved by operating second operation portion 2b is referred to as "a tilt operation" or is simply referred to as "a tilt". In addition, operation ranges (rotation ranges about first axis Ax1) of first and second operation portions 2a and 2b are regulated by a stopper (not shown) provided in grip section 2. In addition, rotary grips may also be used as first and second operation portions 2a and 2b in addition to the lever type operation portions as shown.

FIG. 1 shows an initial state of endoscope 1. In this case, insertion section 5 is linear and the visual field of imaging unit 6a in rigid section 6 is directed forward. From this state, insertion section 5 is bent downward when first operation portion 2a is operated, and imaging unit 6a is tilted downward when second operation portion 2b is operated. Here, given that the bending angle of insertion section 5 is 0° to 90° and the tilt angle of imaging unit 6a is 0° to 90°, the visual field may be enlarged to have a movement range of 0° to 180° by combination of the bending operation and the tilt operation without increasing the bending angle of insertion section 5 (namely, without occupying a large space during bending). That is, in endoscope 1, a direction (imaging direction) in which the tip end of the functional member is directed by bending of insertion section 5 which is linear in the initial state is substantially equal to a direction in which the tip end of the functional member is directed by rotation of the pivoted functional member.

Connection section 3 is provided in front of grip section 2. Connection section 3 is supported on grip section 2 and connected to linear section 4 in the front thereof. As described later, force generated by the operation of first operation portion 2a is transferred to connection section 3 by link member 10, and connection section 3 transfers the force to idle end 5b of insertion section 5 as traction force.

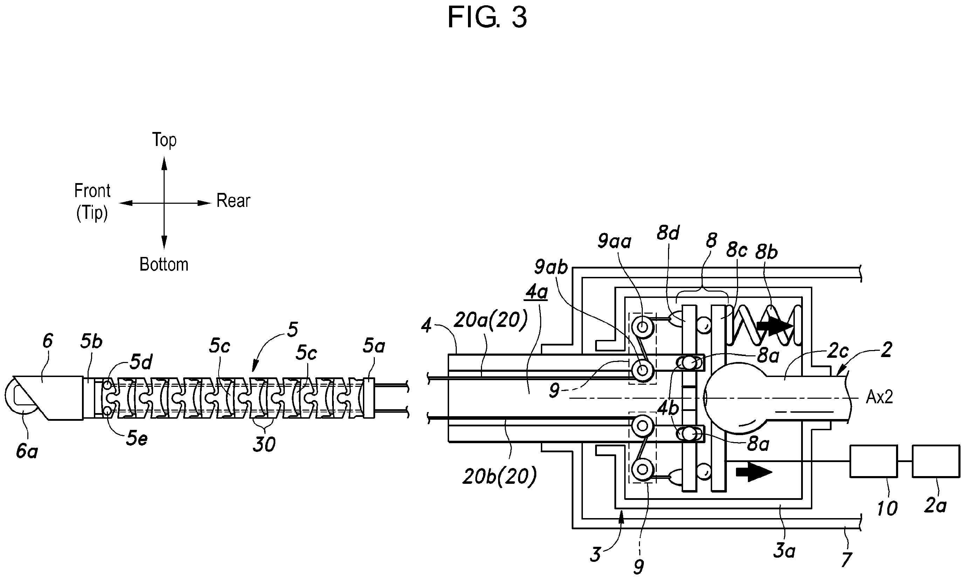

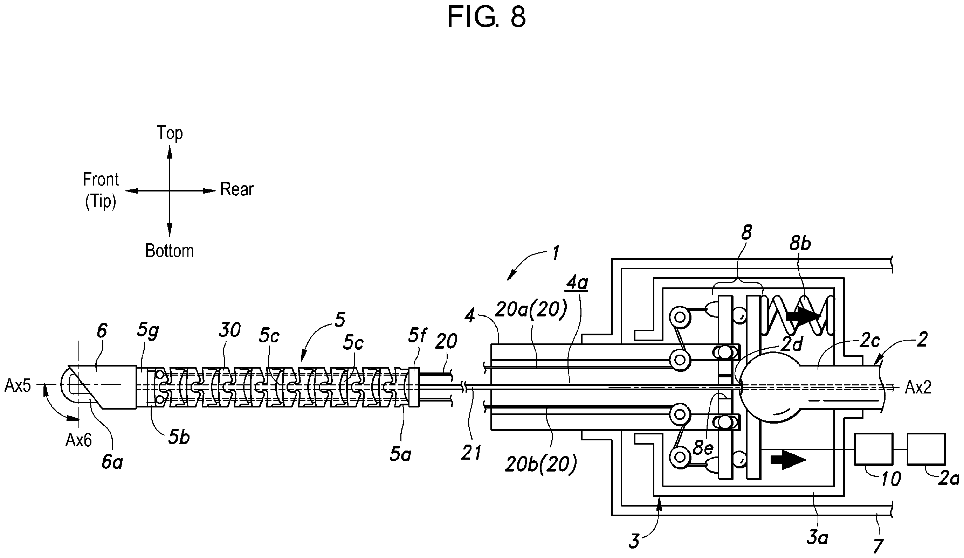

One end of linear section 4 is attached to base end 5a of insertion section 5. Linear section 4 is a cylindrical and linear member having hollow portion 4a (see FIG. 3 and the like) extending in second axis Ax2 direction, and is made of stainless steel herein. Linear section 4 is connected to grip section 2 through connection section 3 to extend forward from grip section 2. Force (hereinafter, the force generated by operating second and first operation portions 2b and 2a, etc. being referred to as "operation force") generated by the operation of second operation portion 2b is converted into rotational force about second axis Ax2 by a gear mechanism provided within grip section 2, and the rotational force is transferred to rigid section 6. In addition, connection section 3 is provided with bearing opening portion 2d (see FIG. 8 and the like) penetrated in second axis Ax2 direction, and the rotational force is directly transferred toward rigid section 6 without passing through connection section 3.

In addition, grip section 2 is connected to video processor 40 which performs image processing with respect to still and moving images obtained by photographing the inside of an observation object (here, a human body), and the images processed by video processor 40 are displayed on display device 41. Meanwhile, endoscope 1 receives power and various control signals from video processor 40 and the imaging is performed by imaging unit 6a at a timing based on the control signals.

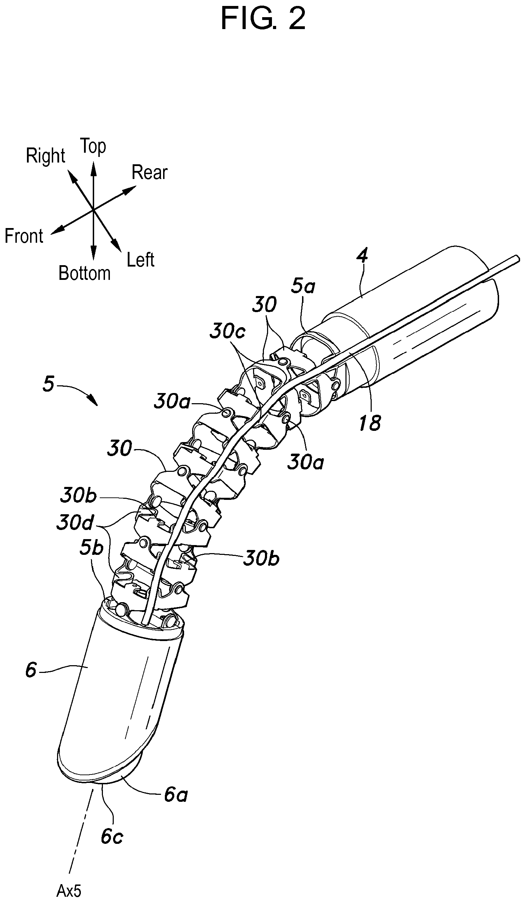

FIG. 2 is a perspective view illustrating a configuration of insertion section 5. As shown in the drawing, insertion section 5 extends from base end 5a to idle end 5b and is configured of plurality of articulated pieces 30 connected between base end 5a and idle end 5b. In the following description, the axis formed by an assembly of plurality of articulated pieces 30 is also referred to as "an axis of insertion section 5" and the direction thereof is referred to as "an axial direction of insertion section 5". Since insertion section 5 is bendable, "the axial direction of insertion section 5" is varied according to the bending direction and the bending angle.

Each of articulated pieces 30 is made of stainless steel herein and is a member having a substantially rectangular shape when viewed from the axial direction of insertion section 5, and all of articulated pieces 30 have the same shapes. Each of articulated pieces 30 has joint portions 30a at left and right (or up and down) symmetrical positions in the front view, and articulated piece 30 is configured to be rotatable about joint portions 30a by a predetermined angle with respect to adjacent articulated piece 30. Idle end 5b of insertion section 5 is configured to be bendable in any direction with respect to base end 5a by shifting joint portions 30a by 90° in the circumferential direction and connecting plurality of articulated pieces 30, when viewed from the axial direction of insertion section 5.

In addition, wire conduction piece 30b formed to be bent in a radial direction from an outer periphery of insertion section 5 is provided at a side in which joint portions 30 are not formed in rectangular articulated piece 30. Control wire 20 (see FIG. 3 and the like) to be described later is provided to extend to a through-hole formed on wire conduction piece 30b.

In addition, first groove portion 30c recessed from an outer surface of articulated piece 30 is provided between joint portion 30a and wire conduction piece 30b in the circumferential direction, namely, is provided in a corner portion of rectangular articulated piece 30 in the front view. First groove portion 30c is provided to extend along the axis of insertion section 5 when viewing insertion section 5 as a whole, and transmission cable 18 made by binding signal lines, power lines, etc. which are drawn from imaging unit 6a so as to transmit image data to video processor 40 is stored in first groove portion 30c. Here, transmission cable 18 is stored so as to be displaceable relative to first groove portion 30c (that is, so as to be slidable along the axis of insertion section 5) since an axial length of insertion section 5 is varied at the outer surface thereof when insertion section 5 is bent. In addition, second groove portion 30d is extensionally provided at a corner portion different from the corner portion of articulated piece 30 in which first groove portion 30c is formed.

For example, a bundle of optical fibers (not shown) through which illumination light is transmitted toward the tip end of rigid section 6 and a water pipe (not shown) through which cleaning solutions are supplied are stored in second groove portion 30d. In addition, separate other groove portions may also be configured at the back side which is not shown in FIG. 2. In a case in which a functional member other than imaging unit 6a is mounted on rigid section 6, when the functional member requires mechanical driving force (for instance, when the functional member is a forceps or an ultrasonic scalpel), a pipe may also be provided to extend to another groove portion and thus the driving force may also be transferred through wires or the like into the pipe. In addition, the outer periphery of insertion section 5 may also be covered by a high flexible cladding material (not shown).

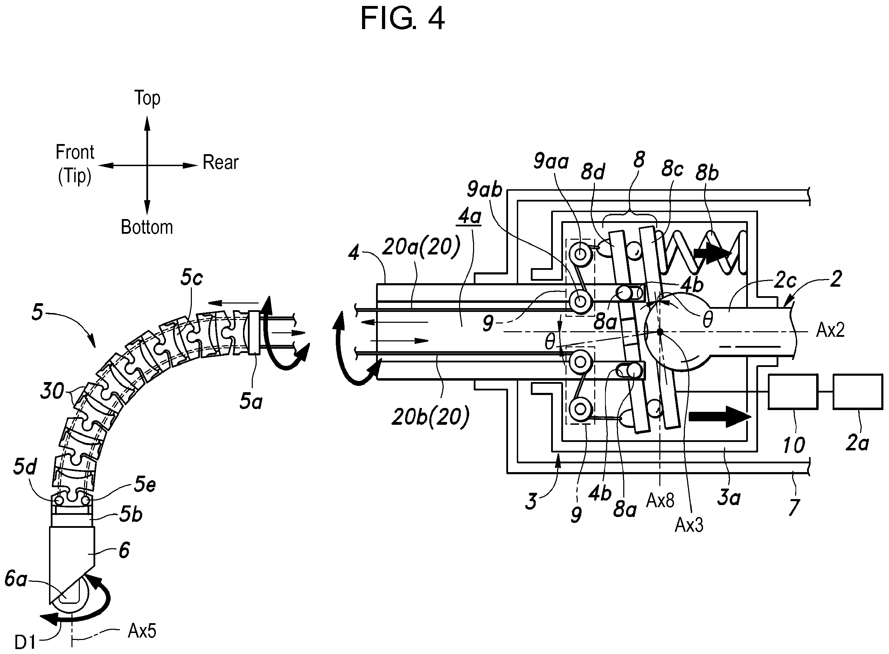

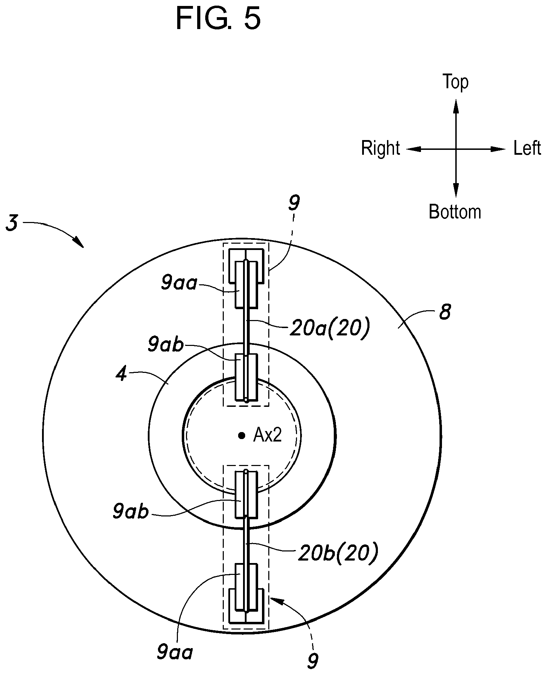

FIGS. 3 and 4 are views for illustrating a basic configuration of connection section 3 and a relationship between a state of connection section 3 and bent state of insertion section 5. FIG. 5 is a view illustrating schematic configurations of traction member 8 and wire guide 9 constituting connection section 3 when viewed from the front. Here, FIG. 3 illustrates a state (an initial state) in which insertion section 5 is linear, and FIG. 4 illustrates a state in which insertion section 5 is bent downward. Hereinafter, a basic configuration in which insertion section 5 is bendable in one direction (in a vertical direction herein) will be described with reference to FIGs. 3 to 5.

Insertion section 3 includes connection section case 3a, and traction member 8 and wire guide 9 provided in connection section case 3a. Spherical bearing 2c is provided at an opposite side of linear section 4 with traction member 8 interposed therebetween, so as to protrude forward from grip section 2. Connection section case 3a is fixed in a state of regulating rotation about second axis Ax2 and movement in the forward and rearward directions in the axial portion of spherical bearing 2c in the rear of connection section case 3a. Connection section case 3a supports linear section 4 so as to be rotatable about second axis Ax2 in the front of connection section case 3a. The axis of linear section 4 always coincides with the axis (second axis Ax2) of spherical bearing 2c by connection section case 3a, namely, is supported by maintaining a coaxial degree.

As shown in FIG. 5, traction member 8 is a disk member having a circular shape in the front view (detailed configuration example being described later), and includes stationary portion 8c provided rearward and rotation portion 8d provided forward as shown in FIG. 3. Stationary portion 8c is supported from the rear by spherical bearing 2c. Spherical bearing 2c regulates movement of entire traction member 8 in the forward and rearward directions and supports traction member 8 (stationary portion 8c) such that traction member 8 (stationary portion 8c) is inclinable in any direction with respect to the plane orthogonal to the axis (second axis Ax2) of linear section 4. Meanwhile, rotation portion 8d is rotatable relative to stationary portion 8c.

Wire guide 9 is a member which is mainly configured by first fixed pulley 9aa and second fixed pulley 9ab. In the basic configuration, two wire guides 9 are fixed at the top and bottom of linear section 4.

As shown in FIGs. 3 and 4, rotation portion 8d of traction member 8 is provided with guide pieces 8a at two upper and lower positions with second axis Ax2 interposed therebetween, and guide pieces 8a are engaged with guide holes 4b formed as slots in the forward and rearward directions in the vicinity of the rear end of linear section 4. Guide pieces 8a and guide holes 4b constitute an engagement mechanism. Traction member 8 is also supported at the rear end of linear section 4 by the engagement mechanism, and is displaceable (inclinable) relative to linear section 4 (second axis Ax2). That is, rotation portion 8d is inclinable with respect to the plane orthogonal to the axis of linear section 4 and is rotated along with linear section 4 in an inclined state.

As described above, linear section 4 maintains the coaxial degree with spherical bearing 2c by connection section case 3a. According to the configuration, since traction member 8 is inclinable by spherical bearing 2c, an inclination direction and inclination angle of traction member 8 are changed within connection section 3 in a state in which a relative positional relationship between grip section 2 (spherical bearing 2c) and linear section 4 at the front and rear of connection section 3 is not changed (that is, in a state in which the coaxial degrees of both are maintained). However, since the engagement structure by guide pieces 8a and guide holes 4b is provided, the inclinable direction of traction member 8 is limited. In addition, as understood from the relationship of FIGs. 3 and 4 herein, only inclination of traction member 8 about third axis Ax3 shown in FIG. 4 is allowable, and in this case, a maximum value of an angle θ is substantially determined by a length of guide hole 4b in the forward and rearward directions thereof in the engagement structure.

As shown in FIG. 3, an upper portion of traction member 8 is normally urged rearward by urging member 8b configured of an elastic body such as a coil spring in the inside of connection section 3, whereas a lower portion of traction member 8 is towed rearward by first operation portion 2a through link member 10 described above.

In addition, starting ends of first and second control wires 20a and 20b are respectively fixed at upper and lower sides on the outer peripheral portion of traction member 8 (hereinafter, these being collectively referred to as "control wires 20"). Twisted yarn of stainless wires or the like may be properly used as control wires 20. Control wires 20 form a first power transfer member, and the starting ends of control wires 20 are towed rearward by traction member 8. In the basic configuration, the starting ends of control wires 20 are fixed at portions (herein, outer peripheral portion in the vertical direction) spaced apart by 180° in the circumferential direction with second axis Ax2 interposed therebetween in the outer peripheral portion of traction member 8. Wire guide 9 is provided in front of traction member 8 so as to correspond to the fixed positions of control wires 20.

Wire guide 9 is fixed to the outer periphery of linear section 4 so as not to be relatively displaced, and is configured of first fixed pulley 9aa provided at the outer peripheral side and second fixed pulley 9ab provided at the inner peripheral side. Control wires 20 first change an extension direction thereof from the outer peripheral side to the inner peripheral side by first fixed pulley 9aa and then change the extension direction from the rear to the front by second fixed pulley 9ab. Control wires 20 the extension direction of which is changed forward by second fixed pulley 9ab are introduced to base end 5a of insertion section 5 in hollow portion 4a of cylindrical linear section 4 and is then introduced to idle end 5b of insertion section 5 via the conduction holes of wire conduction pieces 30b (see FIG. 2) protruding toward the inner side of insertion section 5 in order.

As shown in FIG. 3, a trailing end of first control wire 20a is fixed at first fixed point 5d provided at the top of insertion section 5 in the inner surface of idle end 5b of insertion section 5. Similarly, a trailing end of second control wire 20b is fixed at second fixed point 5e provided at the bottom of insertion section 5.

When operation force is given rearward in the lower portion of traction member 8 by the operation of first operation portion 2a in the initial state shown in FIG. 3, traction member 8 is inclined by the angle θ about third axis Ax3 with respect to the plane orthogonal to second axis Ax2, according to an operation amount of first operation portion 2a. Second control wire 20b is towed rearward according to the inclination of traction member 8, second fixed point 5e is towed at idle end 5b of insertion section 5, and insertion section 5 is finally bent rearward, as shown in FIG. 4. In this case, first control wire 20a connected to first fixed point 5d is drawn out forward according to the bending of insertion section 5.

In addition, a drawn length (hereinafter, referred to as "a traction amount") of each control wire 20 by traction member 8 is determined by the inclination angle of traction member 8 about third axis Ax3 and the distance between the position at which the starting end of control wire 20 is fixed to traction member 8 and third axis Ax3 (to be extent, an intersection point between the plane to which the starting end of control wire 20 is fixed and second axis Ax2). Accordingly, the traction amount is increased by increasing the outer diameter of traction member 8 and thus the bending angle of insertion section 5 may be increased. Since connection section case 3a storing traction member 8 is present outside the body, the size of the outer diameter is not especially limited.

In addition, although the state in which insertion section 5 is not bent is the initial state herein as shown in FIG. 3, a state in which insertion section 5 is bent upward by adjusting tension of urging member 8b may also be set as the initial state. Thereby, insertion section 5 is changed from the upward bent state to the linear state shown in FIG. 3 by the operation of first operation portion 2a, and may be displaced to a downward bent state shown in FIG. 4 by further operating first operation portion 2a.

In addition, in the inside of connection section case 3a, although the upper portion of traction member 8 is urged rearward by urging member 8b in the basic configuration, the upper portion of traction member 8 may also be engaged with link member 10 so as to be formed in a push-pull configuration at the top and the bottom. Thereby, first control wire 20a is towed based on the operation of first operation portion 2a so that insertion section 5 may be bent upward from the initial state shown in FIG. 3.

In addition, insertion section 5 may also be configured so as to autonomously maintain a linear state (or the upward bent state as described above) as the initial state, for example, by interconnecting adjacent articulated pieces 30 using an elastic member (not shown) such as a spring. In this case, when control wires 20 are not towed, the bending direction of insertion section 5 is limited in one direction since insertion section 5 is returned to the initial state by self-elasticity, but at least one of control wires 20 is enough.

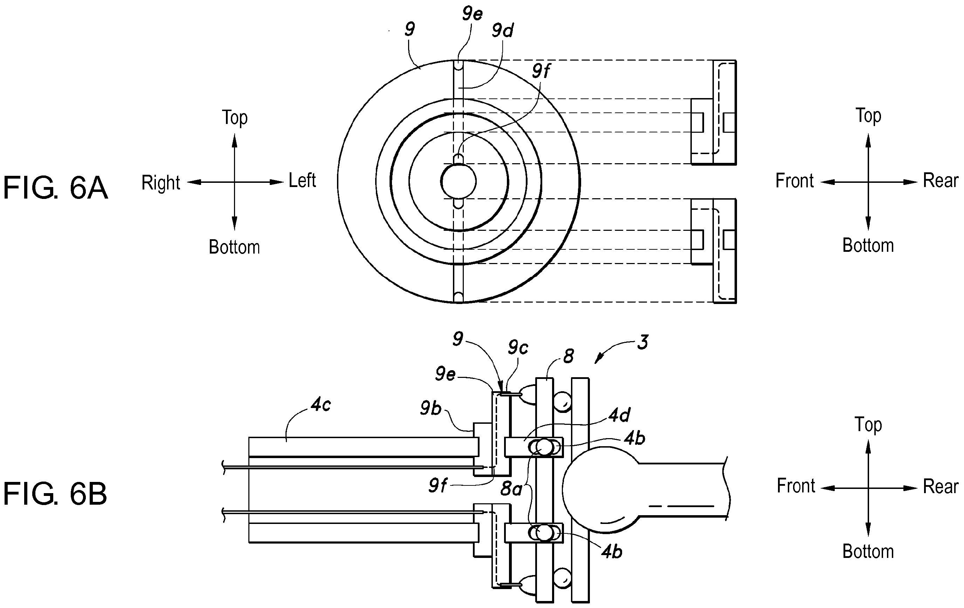

FIGS. 6A and 6B are views for illustrating a first modification example of connection section 3. Although wire guide 9 has first and second fixed pulleys 9aa and 9ab (see FIG. 3 and the like) in the above basic configuration, wire guide 9 is configured as a single member in the first modification example. As shown in the drawings, wire guide 9 is configured as a flange-shaped member having a small diameter portion 9b at the front and a large diameter portion 9c at the rear in the first modification example. Wire guide 9 in the first modification example is preferably made of metal such as stainless steel or ceramic since metallic control wires 20 slide on the surface of wire guide 9.

In the first modification example, wire guide 9 is interposed between two pieces of linear section 4 configured in the forward and rearward directions. That is, linear section 4 is configured of front portion 4c and rear portion 4d. Front portion 4c is fitted into a concentric groove formed on small diameter portion 9b of wire guide 9. Similarly, rear portion 4d is fitted into a groove formed on large diameter portion 9c. Guide holes 4b are formed in the vicinity of a rear end of rear portion 4d of linear section 4 so that guide pieces 8a of traction member 8 are engaged with guide holes 4b.

Guide groove 9d is extensionally provided on an outer peripheral surface and front surface of large diameter portion 9c of wire guide 9. Guide groove 9d extends between large diameter portion 9c and small diameter portion 9b in the inner peripheral side of large diameter portion 9c, and then reaches a front surface of small diameter portion 9b by further extending an inner peripheral surface of small diameter portion 9b in the forward and rearward directions. Control wires 20 are guided by guide groove 9d. In addition, small and large diameter portions 9b and 9c are created as separate members, and are integrated to be wire guide 9 by bonding them. By such a configuration, guide groove 9d may be easily provided at a portion in which large and small diameter portions 9c and 9b overlap each other.

In control wires 20 the starting ends of which are fixed to traction member 8, the respective extension directions of control wires 20 are switched by an outer periphery (first direction switching portion 9e) of the front surface of large diameter portion 9c and an inner periphery (second direction switching portion 9f) of the lower surface of small diameter portion 9b via guide groove 9d. R-chamfering is formed on all of first and second direction switching portions 9e and 9f and control wires 20 are smoothly movable along guide groove 9d. In addition, in order to decrease friction when control wires 20 are moved, fluorine treatment or the like is preferably performed on the surfaces of first and second direction switching portions 9e and 9f so as to increase sliding. Of course, control wires 20 may also be processed to have high sliding.

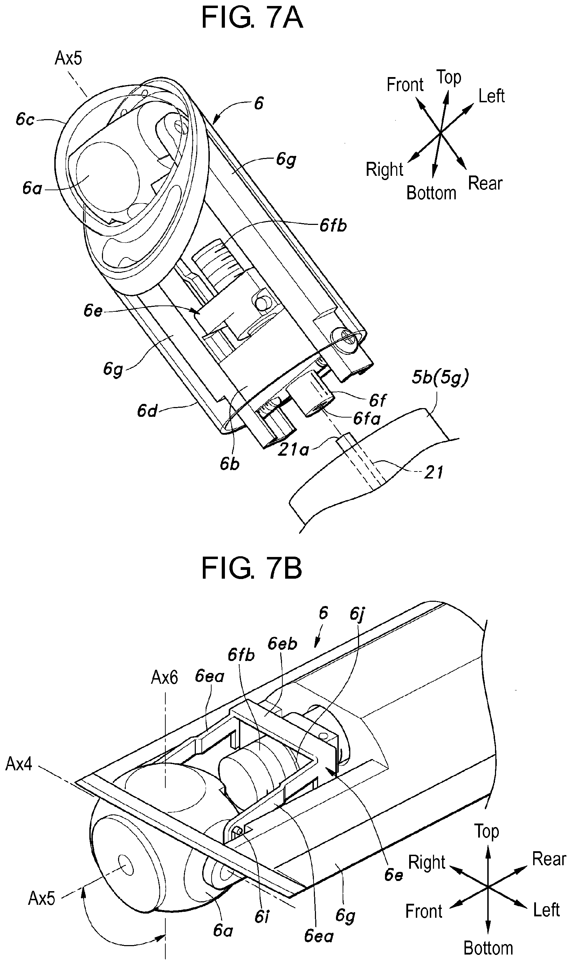

FIGS. 7A and 7B are perspective views illustrating a configuration of rigid section 6 attached to idle end 5b of insertion section 5. Rigid section 6 mainly includes camera support 6b, camera outer block 6d, imaging unit 6a, functional member displacement portion 6e which displaces imaging unit 6a, and coupling portion to be engaged 6f which transfers driving force from the outside to functional member displacement portion 6e.

Camera outer block 6d is a stainless member having a substantially cylindrical shape. In a length of camera outer block 6d in the forward and rearward directions, an upper side thereof is long and a lower side thereof is short. A tip end of camera outer block 6d is formed with a cut surface which is obliquely cut with respect to the forward and rearward directions. The cut surface is provided with transparent dome 6c having a hemispheric shape, and imaging unit 6a is provided in dome 6c.

Imaging unit 6a has an imaging device (not shown) configured of a small charge coupled device (CCD) or a complementary metal-oxide semiconductor (CMOS) and an optical lens (not shown) to focus subject light incident through dome 6c on the imaging device. Imaging unit 6a is pivoted from both left and right by support arm 6g extending in the forward and rearward directions in the left and right of camera support 6b. In addition, imaging unit 6a having such a shape is easily realized, for example, by applying a camera module used in a smart phone or a tablet terminal.

Functional member displacement portion 6e includes engagement portion 6i which is radially spaced from fourth axis Ax4 and provided in imaging unit 6a, drive arms 6ea which are engaged with both sides of imaging unit 6a in engagement portion 6i and extend rearward from engagement portion 6i, and arm support 6eb which supports drive arms 6ea from the rear. Screw hole 6j which is penetrated in the forward and rearward directions is provided at a substantial center of arm support 6eb in the front view, and coupling portion to be engaged 6f is inserted into screw hole 6j.

Coupling portion to be engaged 6f passes through camera support 6b to be exposed from a rear end of camera support 6b, and corner hole 6fa recessed forward is provided at the rear end of camera support 6b. In the inside of rigid section 6, a front portion of coupling portion to be engaged 6f forms lead screw 6fb. Lead screw 6fb is screwed into screw hole 6j formed at arm support 6eb, and drive arms 6ea provided at arm support 6eb move along support arm 6g in the forward and rearward directions by rotating lead screw 6fb (coupling portion to be engaged 6f) about fifth axis Ax5. As such, functional member displacement portion 6e converts rotational motion received by coupling portion to be engaged 6f into linear motion.

Imaging unit 6a engaged with engagement portion 6i rotates about the pivoted axis, namely, about fourth axis Ax4 by support arm 6g, according to forward and rearward movement of drive arms 6ea. As shown in FIG. 7A, coupling portion to be engaged 6f is engaged with coupling engagement portion 21a protruding toward idle end 5b of insertion section 5, and imaging unit 6a is displaced (rotated) in an inclined direction with respect to fifth axis Ax5 by rotating coupling engagement portion 21a about fifth axis Ax5. Thereby, the imaging direction by imaging unit 6a is changed at least between the forward direction (fifth axis Ax5) and the downward direction (sixth axis Ax6), and thus movement of the visual field in the vertical direction, namely, the tilt operation is realized. In addition, a movement amount in the forward and rearward directions is set with respect to a rotation amount by properly setting a pitch of grooves of lead screw 6fb or the like described above, a rotation angle of pivoted imaging unit 6a may be accurately adjusted.

As such, imaging unit 6a itself including the imaging device and the optical lens is configured to rotate about the pivoted axis, but imaging unit 6a may also be configured so as to change an optical path by fixing the imaging device in rigid section 6 and rotating a mirror member (an optical member) pivoted between the imaging device and the optical lens.

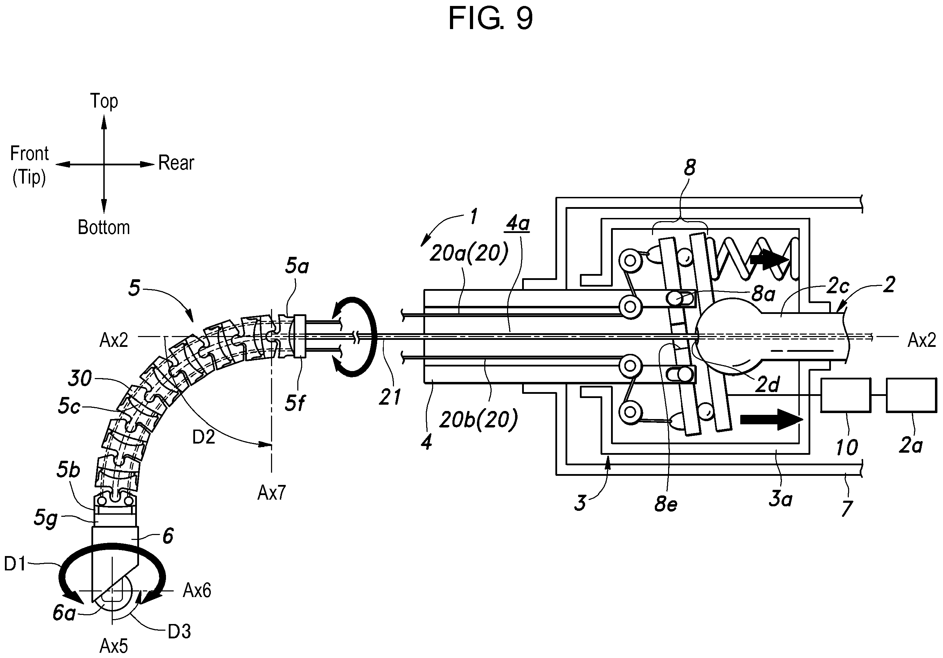

FIGS. 8 and 9 are views for illustrating a configuration of transferring driving force for the tilt operation to rigid section 6. As shown in the drawings, spring joint 21 is extensionally provided between grip section 2 and rigid section 6 as a second power transfer member. In grip section 2, spring joint 21 is mechanically connected to second operation portion 2b (see FIG. 1) through a gear train (not shown) or the like, and spring joint 21 is configured so as to rotate about second axis Ax2 by operating second operation portion 2b. As described above, imaging unit 6a rotates by converting rotation force of lead screw 6fb into linear motion, and in this case, the gear train provided in grip section 2 converts a rotation operation of second operation portion 2b about first axis Ax1 (see FIG. 1) into rotational motion of multiple times. In addition, spring joint 21 is stored in a flexible pipe, and the pipe may also extend between grip section 2 and rigid section 6.

Bearing opening portion 2d and traction member opening portion 8e are respectively formed at the tip end of spherical bearing 2c provided in grip section 2 and the radially central portion of traction member 8 provided in connection section 3. Spring joint 21 extends forward along second axis Ax2 within hollow portion 4a of linear section 4 via bearing opening portion 2d and traction member opening portion 8e. That is, the rotation force transferred by spring joint 21 does not interfere with traction member 8.

Base end-side support member 5f is attached to base end 5a of insertion section 5 and idle end-side support member 5g is attached to idle end 5b. In FIG. 8, all of base end-side support member 5f and idle end-side support member 5g have the substantially same external form as articulated piece 30 in the front view. A plurality of notch portions (not shown) are formed on outer peripheral surfaces of base end-side support member 5f and idle end-side support member 5g in an inner radial direction from an outer periphery. Furthermore, through-holes (not shown) are provided on radially central portions of base end-side support member 5f and idle end-side support member 5g. Control wires 20 pass through the notch portions and spring joint 21 passes through the through-holes.

Even in insertion section 5, spring joint 21 extends along the axis of insertion section 5 within hollow portion 5c of insertion section 5. In order to position spring joint 21 at a radial center of hollow portion 5c even in a state of bending insertion section 5 shown in FIG. 9, an intermediate support member (here, radial size thereof is small so as to be fitted into articulated pieces 30), which is not shown, configured similarly to base end-side support member 5f and idle end-side support member 5g may also be added to all or a portion of articulated pieces 30 so that spring joint 21 passes through a through-hole of the intermediate support member.

Hereinafter, the description will be continuously given with reference to FIG. 7A. The tip end of spring joint 21 protrudes from idle end-side support member 5g. Coupling engagement portion 21a having a square bolt shape is attached to the tip end of spring joint 21. Coupling engagement portion 21a communicates with corner hole 6fa of coupling portion to be engaged 6f provided at the rear end of rigid section 6 so that rotational force of spring joint 21 is transferred to rigid section 6 (functional member displacement portion 6e).

Next, the operation of endoscope 1 will be described with reference to FIGs. 8 and 9. FIG. 8 shows a state in which insertion section 5 is linear in second axis Ax2 direction (that is, rigid section 6 being directed forward) and the visual field of imaging unit 6a provided in rigid section 6 is directed forward. In the state shown in FIG. 8, when the operator or the like operates second operation portion 2b (see FIG. 1) of grip section 2, spring joint 21 rotates according to an operation amount of second operation portion 2b and thus imaging unit 6a pivoted on rigid section 6 rotates so that the visual field of imaging unit 6a is moved downward (in sixth axis Ax6 direction) from the forward direction (fifth axis Ax5 direction). The rotation angle of imaging unit 6a is a maximum of 90°, and the operator or the like may arbitrarily adjust the rotation angle of imaging unit 6a, namely, the tilt angle between fifth axis Ax5 and sixth axis Ax6.

When the operator or the like operates first operation portion 2a of grip section 2 from the state shown in FIG. 8, traction member 8 is inclined according to an operation amount of first operation portion 2a, and thus control wire 20 (here, second control wire 20b) is towed so that insertion section 5 is bent downward (in seventh axis Ax7 direction) from the forward direction (second axis Ax2 direction) as shown in FIG. 9. The bending angle of insertion section 5 is a maximum of 90°, and the operator or the like may arbitrarily adjust the bending angle of insertion section 5 between second axis Ax2 and seventh axis Ax7.

Furthermore, even in a state shown in FIG. 9, the operator or the like may arbitrarily adjust the tilt angle between fifth axis Ax5 and sixth axis Ax6. Here, direction D2 in which insertion section 5 is bent substantially coincides with direction D3 in which pivoted imaging unit 6a is rotated (tilted), and thus the visual field may be moved rearward (in sixth axis Ax6) direction) from the forward direction (second axis Ax2) within a range of 180° by the bending operation of insertion section 5 and the tilt operation of imaging unit 6a. That is, according to the above-mentioned configuration, it is not necessary to uselessly increase the bending angle of insertion section 5 and it may be possible to widely move the visual field even though insertion section 5 is configured to be short. In addition, endoscope 1 may be used in a limited space since the bending angle of insertion section 5 is small. Furthermore, wear of control wires 20 is reduced since the bending angle of insertion section 5 is small, and thus reliability may be maintained for a long time.

In the first exemplary embodiment, the first power transfer member (control wires 20) which transfers operation force generated at base end 5a of insertion section 5, such that insertion section 5 is bent, toward idle end 5b as traction force, and the second power transfer member (spring joint 21) which transfers operation force generated at base end 5a, such that imaging unit 6a, as the functional member, pivoted on rigid section 6 is displaced (rotated), toward idle end 5b as rotational force extend from base end 5a of insertion section 5 to idle end 5b thereof in hollow portion 5c of insertion section 5.

As described above, control wires 20 is disposed along the inner surface of insertion section 5, and spring joint 21 is disposed at the substantially radial center of insertion section 5. Accordingly, even when control wires 20 are operated to bend insertion section 5, the path length of spring joint 21 disposed at the radial center of insertion section 5 is not changed. Therefore, coupling engagement portion 21a (see FIG. 7A) provided at the tip end of spring joint 21 may be always stably engaged with coupling portion to be engaged 6f (see FIG. 7A) provided at the rear end of rigid section 6, and driving force (rotational force) used for the tilt operation of imaging unit 6a may be stably transferred. That is, since the paths of spring joint 21 and control wires 20 in insertion section 5 are fully separated from each other and driving force for movement of the visual field is separately transferred in a different transfer manner from the traction force and the rotational force, interference between the first and second power transfer members may be prevented and the bending operation and the tilt operation may be independently performed.

In addition, although spring joint 21 has been provided as an example of the second power transfer member for transferring the driving force of the tile operation in the above description, the second power transfer member may also be configured as a bar-shaped member having flexibility. In addition, the bar-shaped member may be longitudinally divided into a plurality of division pieces so that the respective division pieces are coupled by joints by adopting the same configuration as insertion section 5 described above.

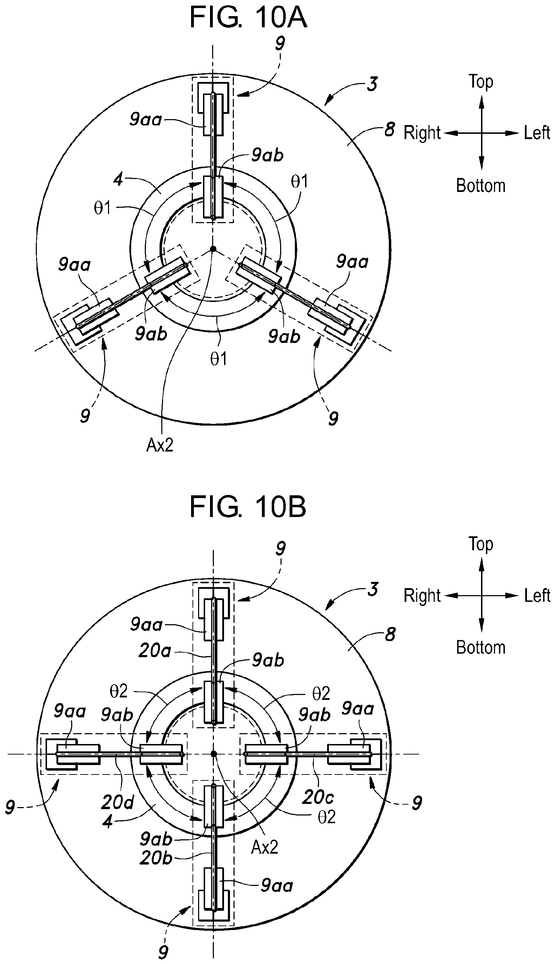

FIG. 10A is a view for illustrating a second modification example of connection section 3. FIG. 10B is a view for illustrating a third modification example of connection section 3. FIGs. 10A and 10B are respective views illustrating traction member 8 and wire guide 9 which constitute connection section 3 when viewed from the front. Although two control wires 20 are connected to traction member 8 constituting connection section 3 in the basic configuration and the first modification example, the number of control wires 20 connected to traction member 8 is increased in the second and third modification examples.

As shown in FIG. 10A, starting ends of three control wires 20 are connected to traction member 8 constituting connection section 3 in the second modification example. Control wires 20 are connected to the outer peripheral side of traction member 8 so as to be spaced apart by an angle θ1 = 120° in the circumferential direction about second axis Ax2. Similarly to being described in the basic configuration, trailing ends of control wires 20 change the extension direction by first and second fixed pulley 9aa and 9ab of wire guide 9, and are then finally connected to the inner surface of insertion section 5 at idle end 5b (see FIG. 3 and the like) of insertion section 5. When insertion section 5 is viewed from the front, the trailing ends of control wires 20 connected to idle end 5b are respectively spaced apart by the angle θ1 = 120° in the circumferential direction about the axis of insertion section 5.

As shown in FIG. 10B, starting ends of four control wires 20 are connected to traction member 8 in the third modification example. Control wires 20 are connected to the outer peripheral side of traction member 8 so as to be spaced apart by an angle θ2 = 90° in the circumferential direction about second axis Ax2. Similarly to the second modification example, trailing ends of control wires 20 are connected to the inner surface of insertion section 5 at idle end 5b (see FIG. 3 and the like) thereof so as to be respectively spaced apart by the angle θ2 = 90° in the circumferential direction about the axis of insertion section 5. In addition, although wire guide 9 is configured of first and second fixed pulley 9aa and 9ab in the second and third modification examples, wire guide 9 may also be configured of a flange-shaped member as in the description of the first modification example, in place of the fixed pulley.

When three or four control wires 20 are used, insertion section 5 may be bent in any direction by selectively towing one control wire 20 or a plurality of control wires 20, and furthermore, the bending angle may also be adjusted by controlling a traction amount of control wire 20. In response to such a configuration, grip section 2 (see FIG. 1) is provided with a third operation portion (not shown). The bending direction and bending angle of insertion section 5 are adjusted and fixed by operating the third operation portion and first operation portion 2a (see FIG. 1) by the operator or the like. That is, grip section 2 including the operation portions and link member 10 (see FIG. 1) includes an inclination setting section which inclines traction member 8 with respect to the plane orthogonal to the axial direction (second axis Ax2) of linear section 4 and sets an inclination direction and inclination angle of traction member 8. The inclination direction or inclination angle of traction member 8 is varied by operating the inclination setting section, and insertion section 5 is bent in any direction by selectively adding traction force to plurality of control wires 20 connected to traction member 8.

Next, the rotation operation of rigid section 6 will be described with reference to FIG. 10B together with FIGs. 3 and 4. In the following description, four control wires 20 shown in FIG. 10B are connected to traction member 8. Only two control wires (first and second control wires 20a and 20b) disposed at the top and bottom are illustrated in FIGs. 3 and 4. A starting end of third control wire 20c shown in FIG. 10B is connected to traction member 8 in the front side of the drawing, and a starting end of fourth control wire 20d is connected to traction member 8 in the back side of the drawing.

As shown in FIGs. 3 and 4, rotation operation section 7 which rotates linear section 4 about second axis Ax2 is fixed to the outer circumference of linear section 4. However, rotation operation section 7 and linear section 4 are not rotated without limitation. That is, grip section2 is provided with a stopper (not shown), and the allowable rotation of rotation operation section 7 is a maximum of one rotation (or half-rotation regarding clockwise or counterclockwise direction) by regulation of the stopper. Since the rotation of linear section 4 is limited as such, transmission cable 18 (see FIG. 2) extending in the axial direction of insertion section 5 is prevented from being excessively twisted.

As described above, stationary portion 8c of traction member 8 is inclinable in any direction with respect to the plane orthogonal to second axis Ax2 by spherical bearing 2c and is fixed so as not to rotate about second axis Ax2. Meanwhile, rotation portion8d of traction member 8 is configured so as to be rotatable about an inclined direction by an angle θ from second axis Ax2. In addition, guide holes 4b forming slots in the forward and rearward directions are provided at the top and bottom in the vicinity of rear end of linear section 4, guide pieces 8a of traction member 8 are guided by guide holes 4b, and traction member 8 is inclinable relative to linear section 4.

As shown in FIG. 10B, since connection section 3 has the same left and right configuration as the upper and lower configuration, traction member 8 is inclinable to the left and the right about eighth axis Ax8 shown in FIG. 4. That is, traction member 8 is configured so as be rotatable about third axis Ax3 and eighth axis Ax8. The inclination direction and inclination angle of traction member 8 are fixed by first operation portion 2a (link member 10) and the third operation portion (not shown) of grip section 2. In addition, FIG. 4 shows a state in which traction member 8 is inclined counterclockwise by an angle θ about third axis Ax3 whereas traction member 8 is not rotated about eighth axis Ax8, and in this case, insertion section 5 is bent downward.

In this state, when rotation operation section 7 fixed to the outer periphery of linear section 4 is rotated about second axis Ax2, linear section 4 is rotated about second axis Ax2 according to the rotation of rotation operation section 7. Such rotation is transferred to rotation portion 8d of traction member 8 through guide holes 4b provided on linear section 4 and guide pieces 8a provided in traction member 8. Since rotation portion 8d is rotatable with respect to stationary portion 8c of traction member 8, rotation portion 8d is rotated about an axis inclined by an angle θ with respect to second axis Ax2.

When rotation portion 8d is rotated, control wires 20 the starting ends of which are fixed to rotation portion 8d and wire guide 9 fixed to linear section 4 are simultaneously rotated according to the rotation of rotation portion 8d. When traction member 8 is rotated, the above-mentioned inclination setting section maintains the inclination direction and inclination angle of stationary portion 8c when viewed from a predetermined direction orthogonal to the axial direction (second axis Ax2) of linear section 4. Thereby, even when rotation portion 8d which is rotatably supported by stationary portion 8c is rotated, the inclination direction and inclination angle of rotation portion 8d are maintained. That is, in connection with the inclination direction and inclination angle of traction member 8, since the state shown in FIG. 4 is always maintained even though rotation operation section 7 is rotated, the respective traction amount of plurality of control wires 20 towed by traction member 8 are changed together with the rotation of rotation portion 8d so that the state in which insertion section 5 is bent downward is secured. That is, when rotation operation section 7 is rotated, idle end 5b of insertion section 5 is rotated in direction D1 about fifth axis Ax5.

Hereinafter, the description will be continuously given with reference to FIG. 9. Rigid section 6 attached to idle end 5b is rotated by the rotation of idle end 5b of insertion section 5. When the initial imaging direction by imaging unit 6a is sixth axis Ax6 direction (rearward direction), the visual field is moved in direction D1 (circumferential direction) about fifth axis Ax5. That is, when insertion section 5 is rotated, imaging unit 6a as the functional member, plurality of control wires 20, and traction member 8 are rotated together with insertion section 5 and traction member 8 changes the traction amount with respect to plurality of control wires 20, so that the bending direction and bending angle of insertion section 5 are maintained. This is an operation corresponding to "panning" in camera work. Hereinafter, the operation of moving the visual field in the circumferential direction is referred to as "panning operation" or simply referred to as "panning".

In addition, when the initial imaging direction is fifth axis Ax5 direction, the imaged image is rotated about the optical axis by the rotation of idle end 5b of insertion section 5. This is an operation corresponding to "roll" in camera work. Hereinafter, the operation of rotating the image about the optical axis is referred to as "roll operation" or simply referred to as "roll". In addition, the operations including "panning operation" and "roll operation" are referred to as "panning operation and the like".

In the first exemplary embodiment, linear section 4 which is not affected by the bending direction and bending angle of insertion section 5 is rotated, the panning operation and the roll operation are performed through an intuitive operation, the outer diameter of rotation operation section 7 described above is greater than that of linear section 4, and the operations are performed by smaller force, so that operability is improved.

Rigid section 6 is rotated when the panning operation and the like are performed, but in this case, rigid section 6 is also rotated relative to spring joint 21 extending along the axis of insertion section 5. For this reason, strictly speaking, the panning operation and the like generate rotational force equivalent to the rotation of spring joint 21. However, lead screw 6fb is required to rotate multiple times in order to rotate pivoted imaging unit 6a (see FIGs. 7A and 7B) described above. Furthermore, since the rotation of rigid section 6 by the panning operation and the like is limited to one rotation at most, the displacement of the tilt angle by the panning operation and the like is not greatly problematic.

However, when the secondary variation of the tilt angle is problematic, a power cut-off section (not shown) which interrupts power transfer by spring joint 21 may also be provided in rigid section 6 or grip section 2 (see FIG. 1), or between rigid section 6 and grip section 2. Specifically, an electromagnetic clutch (not shown) may also be interposed between gear trains configured in grip section 2 and a switch, a touch sensor for detecting a variation in capacitance, or the like may also be provided in rotation operation section 7 (see FIG. 1), as the power cut-off section. Through such a configuration, the rotational force of spring joint 21 may be cut off when the operation by the operator or the like reaches rotation operation section 7.

As such, endoscope 1 of the first exemplary embodiment enables insertion section 5 to be bent in any direction within the body cavity or the like and imaging unit 6a provided at idle end 5b of insertion section 5 to perform the tilt operation and the panning operation and the like. Thereby, a degree of freedom of visual field operation by the operator may be significantly improved and endoscope 1 may be applied to various surgical methods. Since all operations such as bending, tilt, panning, and roll may be performed with the hands of the operator or the like, the surgery or the like may be performed with more safety.

In the surgery, surgical instruments such as a forceps and a laser scalpel in addition to endoscope 1 are inserted into the body cavity. However, due to a positional relationship between endoscope 1 and the other instruments (for example, a case of a positional relationship in which the tip end of rigid section 6 of endoscope 1 faces the tip end of the laser scalpel), a movement direction of the laser scalpel does not sometimes coincide with the direction of the image captured by endoscope 1. According to the first exemplary embodiment, when the imaging direction by imaging unit 6a is set as fifth axis Ax5 direction in FIG. 9, the rotation (top-bottom inversion) of the image about the optical axis may be performed by rolling rigid section 6. Accordingly, the operation direction of the other instrument may always coincide with the top and bottom (left and right) at the image, and thus safety in the surgery or the like may be secured. In addition, the top-bottom inversion (vertical inversion of 180°) may cope by simple image processing. However, when the rotation angle of the image is arbitrarily set, pixels are generated by interpolation in the image processing. For this reason, resolution is deteriorated, particularly, when the number of pixels of the imaging device is small. According to endoscope 1 of the first exemplary embodiment, resolution is not deteriorated since imaging unit 6a itself is rolled.

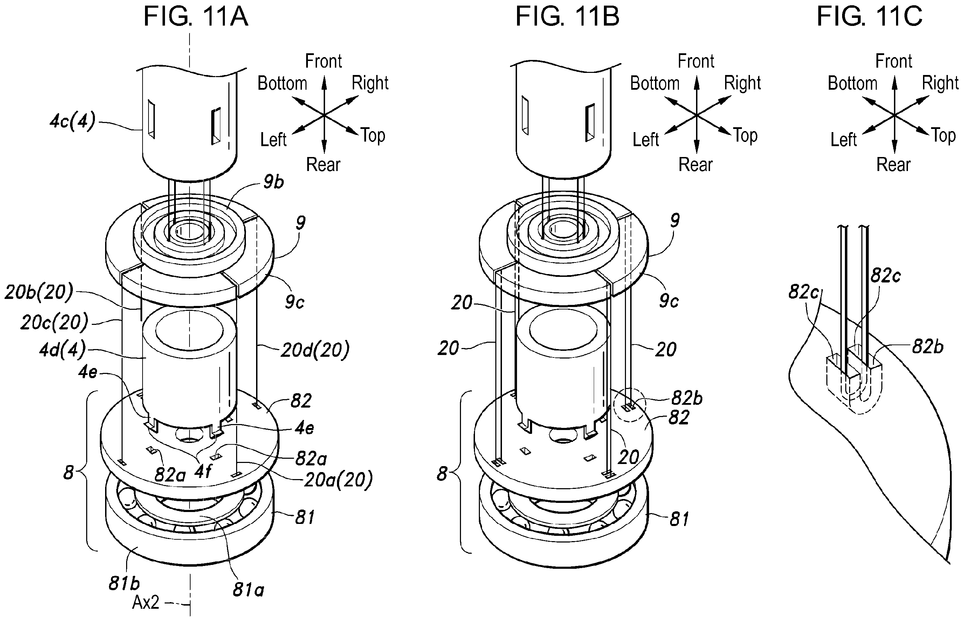

FIG. 11A is an exploded perspective view illustrating a specific configuration of connection section 3. FIG. 11B is a view for illustrating a configuration of enlarging a traction amount. FIG. 11C is a view for illustrating a principal part in FIG. 11B. FIGs. 11A, 11B and 11C show a specific example of the configuration shown in FIG. 10B. However, in FIGs. 11A, 11B and 11C, the flange-shaped member described using FIGs. 6A and 6B is used as wire guide 9.

As shown in FIG. 11A, traction member 8 includes bearing 81 and traction plate 82. Bearing 81 is a so-called a ball bearing which stores metal balls between inner ring 81a and outer ring 81b.

The flange-shaped member (not shown) is fitted beyond inner ring 81a so as not to be rotatable with respect to inner ring 81a, and is supported by spherical bearing 2c (see FIG. 3 and the like) protruding forward from grip section 2. In addition, the flange-shaped member is prevented from rotating at an axial portion of spherical bearing 2c, and thus inner ring 81a is supported so as to be inclinable and not rotatable with respect to spherical bearing 2c. The flange-shaped member includes a large diameter portion (not shown) protruding in the outer radial direction in the rear of a fitted portion with inner ring 81a. Link member 10 (see FIG. 1) constituting the above-mentioned inclination setting section is connected to the large diameter portion, an inclination direction and inclination angle of bearing 81 with respect to spherical bearing 2c are varied by the operation of link member 10.

Meanwhile, traction plate 82 having a cap shape is fixed to outer ring 81b of bearing 81, and is provided so as to be rotatable with respect to inner ring 81a of bearing 81. Inner ring 81a of bearing 81 corresponds to stationary portion 8c of traction member 8 shown in FIGs. 3 and 4, and outer ring 81b of bearing 81 and traction plate 82 correspond to rotation portion 8d.

The starting ends of first to fourth control wires 20a to 20d are fixed to the outer peripheral side of traction plate 82 in each of vertical and horizontal directions, and engagement hole 82a which passes through traction plate 82 in the forward and rearward directions thereof is provided at an intermediate position directed in the inner radial direction from each fixed position of control wires 20. In addition, engagement hole 82a corresponds to guide piece 8a shown in FIGs. 3 and 4.

Here, in the rear end of rear portion 4d of linear section 4, engagement piece 4e protrudes rearward at a position corresponding to engagement hole 82a. Engagement piece 4e has engagement claw 4f which radially protrudes in the rear end thereof. In addition, engagement piece 4e and engagement claw 4f correspond to guide holes 4b shown in FIGs. 3 and 4. Engagement piece 4e is inserted into engagement hole 82a of traction plate 82, and rear portion 4d is stopped from traction plate 82 by engagement claw 4f. However, rear portion 4d of linear section 4 and traction plate 82 are relatively displaced since engagement piece 4e has a predetermined stroke in the forward and rearward directions and idling is present between the base portion of engagement piece 4e and engagement claw 4f provided in the rear end thereof.

Large diameter portion 9c of wire guide 9 is fixed to the front end of rear portion 4d of linear section 4, and small diameter portion 9b of wire guide 9 is fixed to the rear end of front portion 4c of linear section 4. By forming connection section 3 in such a manner, traction member 8 may be inclined with respect to the plane orthogonal to second axis Ax2. Control wires 20 the starting ends of which are fixed to traction plate 82 are introduced into hollow portion 4a (see FIG. 3 and the like) of linear section 4 via wire guide 9, and traction force generated by inclining traction member 8 is transferred toward insertion section 5 (see FIG. 3 and the like). As shown in the drawings, all of bearing 81, traction plate 82, and wire guide 9 are formed with openings at the radial center, and spring joint 21 (see FIG. 8 and the like) described above is inserted into the openings.

In the configuration shown in FIG. 11A, the starting ends of control wires 20 are fixed to the outer peripheral side of traction plate 82, and a movement amount in the forward and rearward directions in displacement on the outer circumference of traction plate 82 (as shown in FIG. 4, traction member 8 rotating about third axis Ax3 and eighth axis Ax8) is the traction amount of control wire 20 as it is by inclining traction plate 82. Since the bending angle of insertion section 5 is determined by the traction amount, the traction amount need be increased if the bending angle is increased.

As shown in FIG. 11B, traction plate 82 is provided with wire relay portion 82b corresponding to each control wire 20. The starting ends of control wires 20 are fixed to the outer peripheral side of the rear surface (back surface) of wire guide 9, and control wires 20 are directed rearward from wire guide 9 (large diameter portion 9c) as a starting point and then directed forward by reversing the extension direction 180 degrees by wire relay portion 82b in traction plate 82. Subsequently, control wires 20 are again drawn from the outer peripheral surface of wire guide 9 to extend to hollow portion 4a (see FIG. 3 and the like) of linear section 4 (front portion 4c) through the above-mentioned path. That is, control wires 20 reciprocate once between traction plate 82 and wire guide 9,

As shown in FIG. 11C, wire relay portion 82b is configured by two through-holes 82c and 82c penetrating traction plate 82 in the forward and rearward direction, and control wires 20 are inserted into through-holes 82c and 82c and thus substantially connected to traction plate 82. That is, even in the configuration of FIG. 11B, the starting ends of control wires 20 are towed rearward by traction member 8. In addition, in order to smoothly slide control wires 20, intermediate portions of through-holes 82c and 82c in the rear surface of traction plate 82 are preferably configured to be curved. High sliding films for prevention of wear may also be applied to the curved portions. In addition, wire relay portion 82b may also be configured by a pulley.

By such a configuration, wire relay portion 82b provided in inclinable traction plate 82 substantially functions as a movable pulley. Accordingly, a traction amount by control wires 20 when traction plate 82 having the movable pulley is inclined doubles compared to the configuration of FIG. 11A. By providing such an enlarged displacement mechanism, the bending angle of insertion section 5 may be enlarged. A configuration of having a single movable pulley with respect to one control wire 20 is illustrated in FIG. 11B,, but a plurality of movable pulleys are provided in traction plate 82 with respect to one control wire 20 and control wire 20 may also extend so as to reciprocate multiple times between traction plate 82 and wire guide 9. In this case, through-holes 82c and 82c shown in FIG. 11C are also provided at wire guide 9 (large diameter portion 9c) and control wire 20 is inserted into through-holes 82c and 82c. Thereby, the traction amount of control wire 20 may be significantly increased and thus the bending angle of insertion section 5 may be significantly increased.

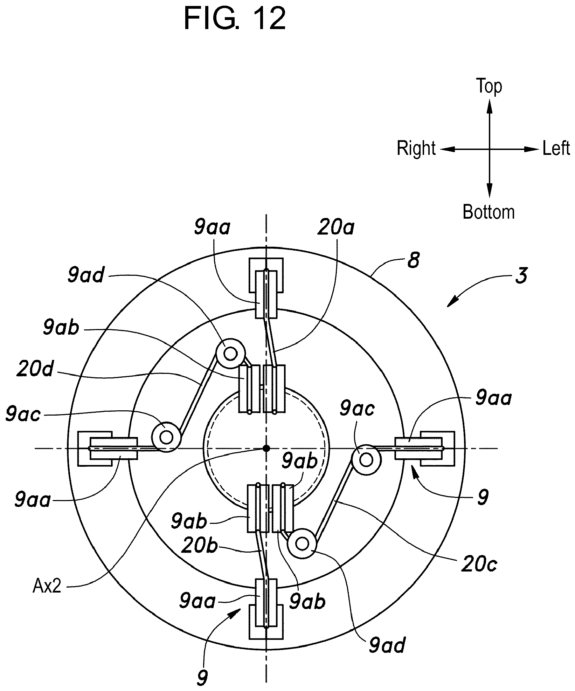

FIG. 12 is an explanatory view illustrating a fourth modification example of connection section 3. In FIG. 12, traction member 8 and wire guides 9 constituting connection section 3 are viewed from the front side. In the third modification example illustrated in FIG. 10B, each wire guide 9 provided in connection section 3 is configured of a set of first fixed pulley 9aa and second fixed pulley 9ab, and four sets of wire guides 9 are provided so as to be separated from one another by 90° in the circumferential direction with second axis Ax2 as the center. In the fourth modification example, the configurations of wire guides 9 arranged in the vertical direction are the same as those of the third modification example, but wire guides 9 arranged in the horizontal direction include third fixed pulleys 9ac and fourth fixed pulleys 9ad allowing the routing direction of control wire 20 to be changed on the surfaces orthogonal to second axis Ax2.

In wire guides 9 arranged in the horizontal direction, third control wire 20c and fourth control wire 20d are routed in order of first fixed pulley 9aa, third fixed pulley 9ac, fourth fixed pulley 9ad, and second fixed pulley 9ab with traction member 8 as the starting point, and two control wires 20 are stretched to the front side as a pair such that third control wire 20c is brought into close contact with second control wire 20b and fourth control wire 20d is brought into close contact with first control wire 20a in the end.

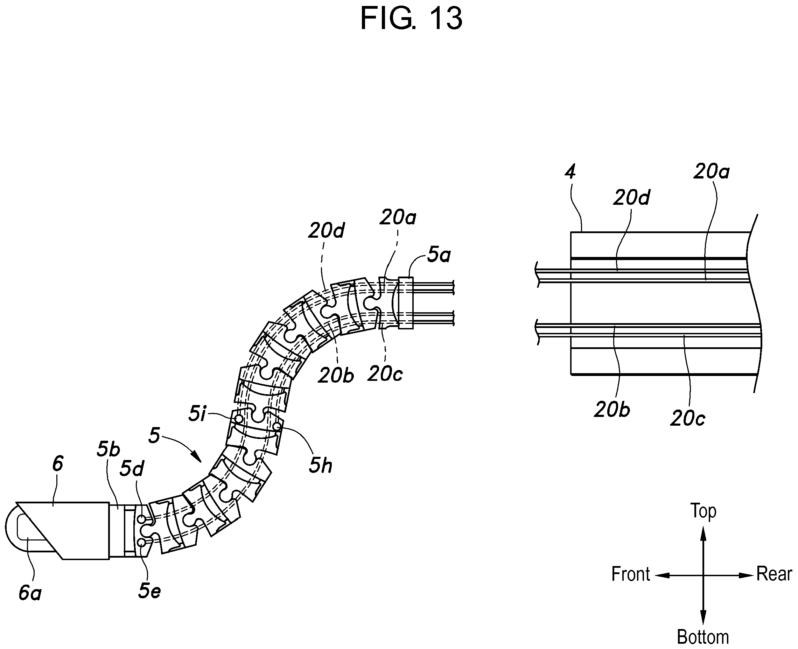

FIG. 13 is an explanatory view illustrating the bent state of insertion section 5 in the fourth modification example. In the fourth modification example, positions of terminals to be fixed onto the inner surface of insertion section 5 are set to be different from each other in regard to one pair of control wires 20. That is, in regard to the pair configured of first control wire 20a and fourth control wire 20d, the terminal of first control wire 20a is fixed to first fixed point 5d which is provided in the vicinity of idle end 5b on the inner surface of insertion section 5 and the terminal of fourth control wire 20d is fixed to the midpoint (fourth fixed point 5i) provided in the portion between base end 5a and idle end 5b on the inner surface of insertion section 5. The length from base end 5a to the midpoint may be approximately a half of the length from base end 5a to idle end 5b. Similarly, the terminal of second control wire 20b constituting another pair is fixed to second fixed point 5e on idle end 5b side and the terminal of third control wire 20c is fixed to third fixed point 5h in the portion between base end 5a and idle end 5b.

Here, for example, when third control wire 20c is pulled, the second half of insertion section 5 is bent downward. At this time, since the total length of first control wire 20a and second control wire 20b which have not been pulled in hollow portion 5c of insertion section 5 is maintained to be constant, the first half of insertion section 5 is bent forward concurrently with the traction of third control wire 20c so that insertion section 5 is bent as illustrated in FIG. 13. In this state, when first control wire 20a is more pulled, only the first half of insertion section 5 is bent upward. In this manner, in the fourth modification example, it is possible to observe an affected area from the front by bending insertion section 5 which is inserted in parallel with the affected area in an S shape.

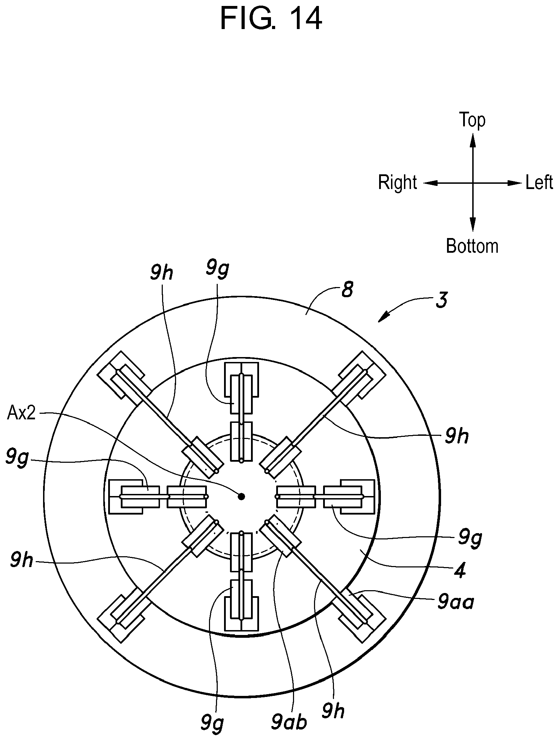

FIG. 14 is an explanatory view illustrating a fifth modification example of connection section 3. In FIG. 14, traction member 8 and wire guide 9 constituting connection section 3 are viewed from the front side. In the fifth modification example, wire guide 9 is configured of first wire guide groups 9g and second wire guide groups 9h. First wire guide groups 9g have the same configurations as those of four wire guides 9 illustrated in FIG. 10B. Second wire guide groups 9h are provided in a state in which second wire guide groups 9h are rotated by 45° in the circumferential direction with respect to first wire guide groups 9g with second axis Ax2 as the center.

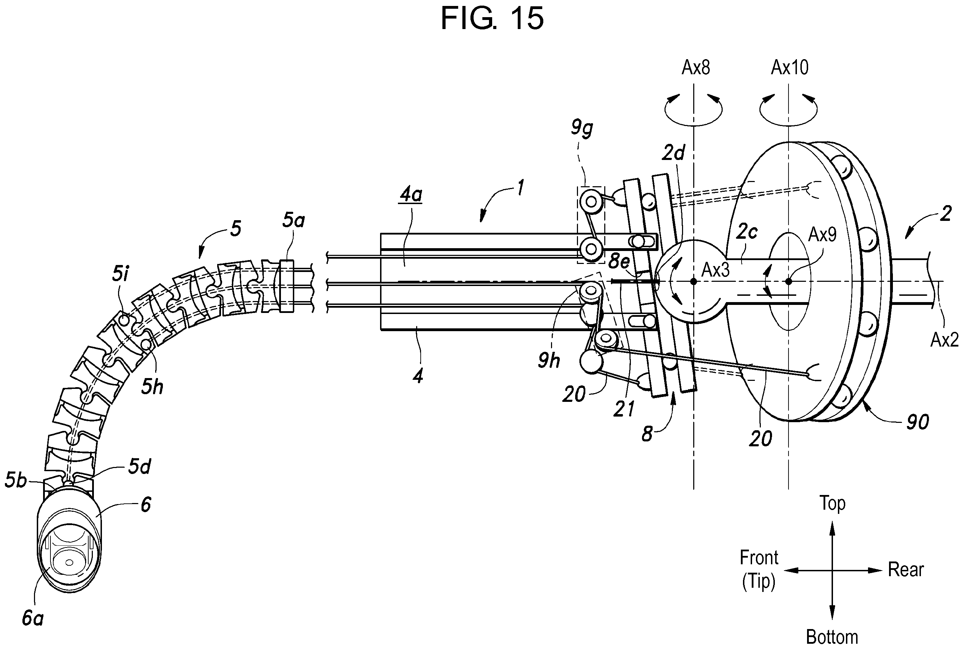

FIG. 15 is an explanatory view illustrating a schematic configuration of endoscope 1 and the bent state of insertion section 5 in the fifth modification example. In the fifth modification example, endoscope 1 is provided with second traction member 90 in addition to the above-described traction member 8. Traction member 8 is configured to be inclinable using third axis Ax3 and eighth axis Ax8 as axes in the same manner as the configuration described as the third modification example. Second traction member 90 is supported by second spherical bearing (not illustrated) provided on the same axis (second axis Ax2) as spherical bearing 2c and configured to be inclinable using ninth axis Ax9 and tenth axis Ax10 as axes. In order to avoid the drawing from becoming complicated, FIG. 15 illustrates only a part of first wire guide groups 9g and second wire guide groups 9h, and a part of control wire 20.

Control wire 20 whose starting end is fixed to traction member 8 is guided to hollow portion 4a of linear section 4 by first wire guide group 9g to be stretched to insertion section 5, and then fixed onto the inner surface of insertion section 5 in the intermediate position of base end 5a and idle end 5b of insertion section 5. In FIG. 15, as a position to which the terminal of control wire 20 is fixed, only two sites of third fixed point 5h and fourth fixed point 5i are described, but the terminal thereof is practically fixed to four sites separated from one another by 90° in the circumferential direction in the intermediate position. Control wire 20 whose starting end is fixed to second traction member 90 is similarly stretched to insertion section 5 by second wire guide group 9h, and then fixed onto the inner surface of insertion section 5 in idle end 5b of insertion section 5. In FIG. 15, as a position to which the terminal of control wire 20 is fixed, only first fixed point 5d is described, but the terminal thereof is practically fixed to four sites.

With such a configuration, it is possible to bend the second half of insertion section 5 by adjusting the inclination direction and the inclination angle of traction member 8 using third axis Ax3 and eighth axis Ax8 as the rotation center and to bend the first half of insertion section 5 by adjusting the inclination direction and the inclination angle of second traction member 90 using ninth axis Ax9 and tenth axis Ax10 as the rotation center. In this manner, it is possible to realize a complicated operation of bending the second half of insertion section 5 downward and bending the first half leftward.

Even in the fifth modification example, traction member 8 and second traction member 90 are configured to be rotatable together with rotation operation section 7 (see FIG. 4), and linear section 4 and insertion section 5 are rotated when an operator or the like rotates rotation operation section 7 and then imaging unit 6a performs a pan operation and a roll operation as described above with reference to FIG. 4.

Bearing opening portion 2d is provided in spherical bearing 2c and traction member opening portion 8e is provided in traction member 8. Spring joint 21 is stretched to the front side through these opening portions and connected to rigid section 6. As specifically described above with reference to FIGs. 7A, 7B, and 9, when the operator or the like operates second operation section 2b (see FIG. 1), imaging unit 6a (see FIGs. 7A and 7B or the like) provided in rigid section 6 performs a tilt operation. In this manner, since the degree of freedom of insertion section 5 in the bent direction is increased and the pan operation, the roll operation and the tilt operation can be performed on rigid section 6 in the fifth modification example, the range to which endoscope 1 is applied is further enlarged.

### [SECOND EXEMPLARY EMBODIMENT]

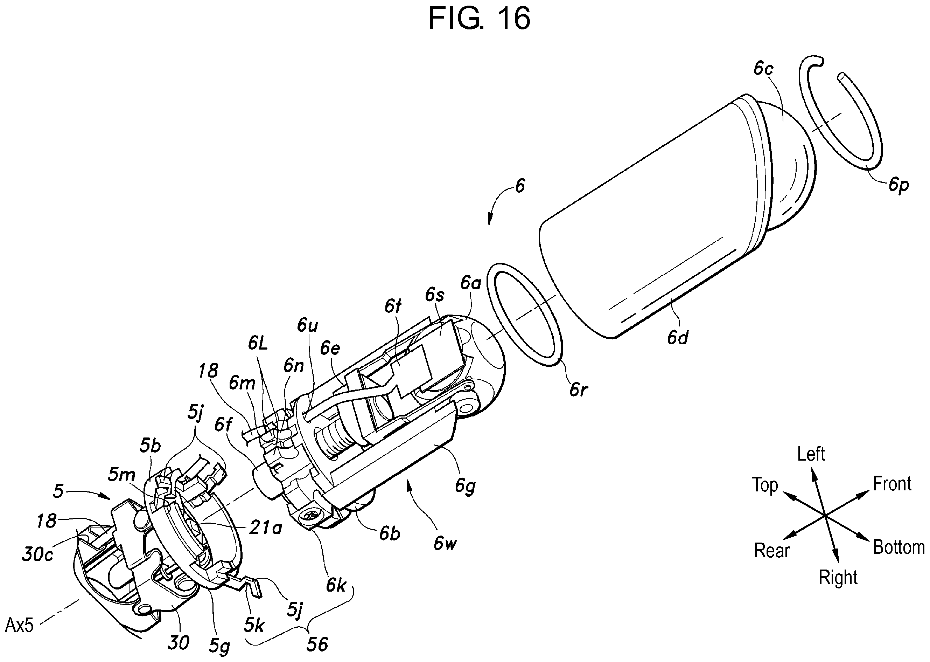

FIG. 16 is a perspective view illustrating the configuration of first connection section 56 connecting insertion section 5 and rigid section 6 in endoscope 1 according to the second exemplary embodiment of the present invention. Since insertion section 5 and rigid section 6 have the same configurations as those described in the first embodiment (see the description with reference to FIGs. 7A and 7B) except that first connection section 56 is included, redundant description will not be repeated.

In the second exemplary embodiment, rigid section 6 of endoscope 1 is configured to be detachable with respect to insertion section 5 on idle end 5b side of insertion section 5, and rigid section 6 and transmission cable 18 drawn from rigid section 6 are considered as disposable objects. Rigid section 6 is connected to insertion section 5 through first connection section 56.

Rigid section 6 is mainly configured of camera portion 6w, camera contour 6d whose distal end includes dome 6c, and O-ring 6r which is a seal member. In a process of producing rigid section 6, O-ring 6r is mounted on the outer periphery of camera portion 6w, and then camera contour 6d is attached from the front side. Grooves (not illustrated) are provided on the inner surface of camera contour 6d in the longitudinal direction and are guided to support arm 6g. In this manner, positioning of imaging unit 6a pivotally supported by support arm 6g and dome 6c obliquely attached to the distal end of rigid section 6 is performed. Camera portion 6w and camera contour 6d are watertightly sealed and fixed by injecting an adhesive from the backside of camera support 6b.

Driving substrate 6s is fixed to imaging unit 6a in camera portion 6w. Driving substrate 6s relays a control signal transmitted from video processor 40 (see FIG. 1) through transmission cable 18 and flexible cable 6t and generates a timing signal that drives an imaging device (not illustrated) placed on imaging unit 6a based on the control signal. Driving substrate 6s temporarily relays image data output from the imaging device and then the image signal is output toward video processor 40 through flexible cable 6t and transmission cable 18. A relay substrate (not illustrated) is stored in base portion 6k of camera support 6b, flexible cable 6t drawn into base portion 6k from cable insertion section 6u is connected to transmission cable 18 through the relay substrate, and transmission cable 18 is drawn out toward the backside of external rigid section 6 from the lateral side of base portion 6k.

Hereinafter, the configuration of first connection unit 56 and procedures of attaching or detaching rigid section 6 to or from idle end 5b side of insertion section 5 will be described. First mobile engaging claws 5j separated from one another at equal intervals are provided on three sites of the outer edge of support member 5g of insertion section 5 on idle end side in the circumferential direction with fifth axis Ax5 as the center. The base portions of first mobile engaging claws 5j are axially supported by support member 5g on the idle end side and the distal ends thereof are displaced (opened and closed) in the radial direction of insertion section 5 (in FIG. 16, an opened state is illustrated). Concave portion 5k that recesses in the radial direction is formed on the distal end side of first mobile engaging claw 5j.

A plurality of projections 6L are provided on the outer periphery of base portion 6k of rigid section 6 in the circumferential direction. Engaging grooves 6m that recess from the outer periphery in the radial direction are disposed between adjacent projections 6L in the longitudinal direction. Engaging grooves 6m are provided in positions corresponding to first mobile engaging claws 5j (in FIG. 16, engaging groove 6m appears in only one upper site, but engaging grooves are practically provided in three sites). In projections 6L, the front side of projections 6L is cut out to be recessed from the outer periphery in the radial direction, and first groove 6n for fixation intersecting engaging groove 6m is disposed so as to move around base portion 6k. However, first groove 6n for fixation is fragmentally provided in the circumferential direction as illustrated in the figure.

Hereinafter, procedures of mounting rigid section 6 on idle end 5b of insertion section 5 will be described. When base portion 6k is brought into contact with support member 5g on the idle end side by aligning engaging groove 6m of base portion 6k in accordance with the position of first mobile engaging claw 5j of support member 5g on the idle end side by a user or the like, the distal end of first mobile engaging claw 5j rotates in the inner diameter direction and first mobile engaging claw 5j is stored in engaging groove 6m.

When first mobile engaging claw 5j is stored in engaging groove 6m, the depths of concave portion 5k and engaging groove 6m are set such that the outer surface of concave portion 5k of first mobile engaging claw 5j becomes substantially flush with the outer surface of first groove 6n for fixation which is fragmentally provided in base portion 6k of camera support 6b in the circumferential direction. Rigid section 6 is temporarily mounted on insertion section 5 by the rear portion of camera support 6b being fitted into the front side of annular shoulder surface 5m formed on the outer peripheral edge of support member 5g on the idle end side.

When C-ring 6p made of stainless steel serving as a fixing member is pressed from the front side with respect to temporarily mounted rigid section 6 by the user or the like, the diameter of C-ring 6p is increased and pushed to the backside of camera contour 6d, and C-ring 6p reaches the position of first groove 6n for fixation in the end. C-ring 6p fitted into first groove 6n for fixation enters concave portion 5k of first mobile engaging claw 5j at the same time, and first mobile engaging claw 5j is interposed between the outer periphery of first groove 6n for fixation and C-ring 6p so that rigid section 6 is mounted and fixed onto idle end 5b of insertion section 5.

Corner bolt-shaped shaft coupling section 21a that projects on the distal end side of support member 5g on the idle end side is fitted into angular hole 6fa (see FIG. 7A) of shaft-coupled section 6f that projects from the rear end of camera support 6b by performing the above-described processes. In this manner, it is possible to connect corner bolt-shaped shaft coupling section 21a with angular hole 6fa of shaft-coupled section 6f on the coaxial line and to transmit the rotational force which is provided for displacement of a functional member (imaging unit 6a (see FIGs. 7A and 7B)) placed on rigid section 6.

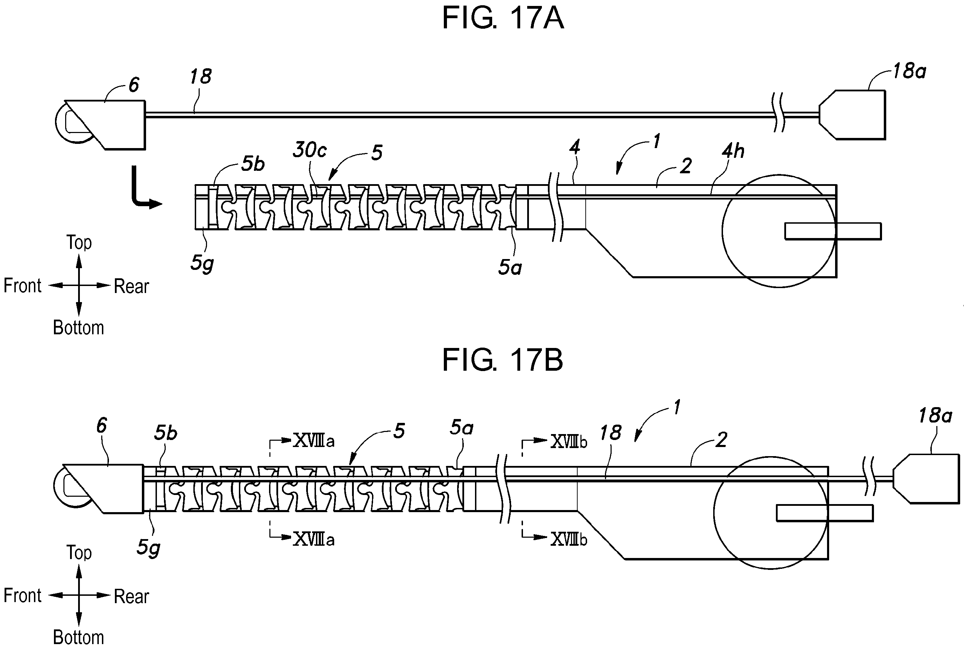

After rigid section 6 is mounted on idle end 5b of insertion section 5, transmission cable 18 is pushed into first groove portion 30c (see FIGs. 17A and 17B) provided in the outer periphery of joint piece 30 constituting insertion section 5 by the user or the like. In this manner, transmission cable 18 is arranged rearward along insertion section 5. Transmission cable 18 which is a disposable object is mounted on endoscope 1 through the above-described processes. C-ring 6p is exposed to the outside in this state, but camera contour 6d (except the portion of dome 6c) and insertion section 5 may be coated with a coating material made of a resin with high elasticity if necessary.

Hereinafter, procedures of detaching rigid section 6 from idle end 5b side of insertion section 5 will be described. First, transmission cable 18 is removed from joint piece 30 by the user or the like. Next, C-ring 6p which is exposed in rigid section 6 is cut using nippers or the like. In this manner, engagement of first groove 6n for fixation of rigid section 6 and first mobile engaging claw 5j provided in support member 5g on the idle end side is released, so that rigid section 6 can be easily removed from insertion section 5.

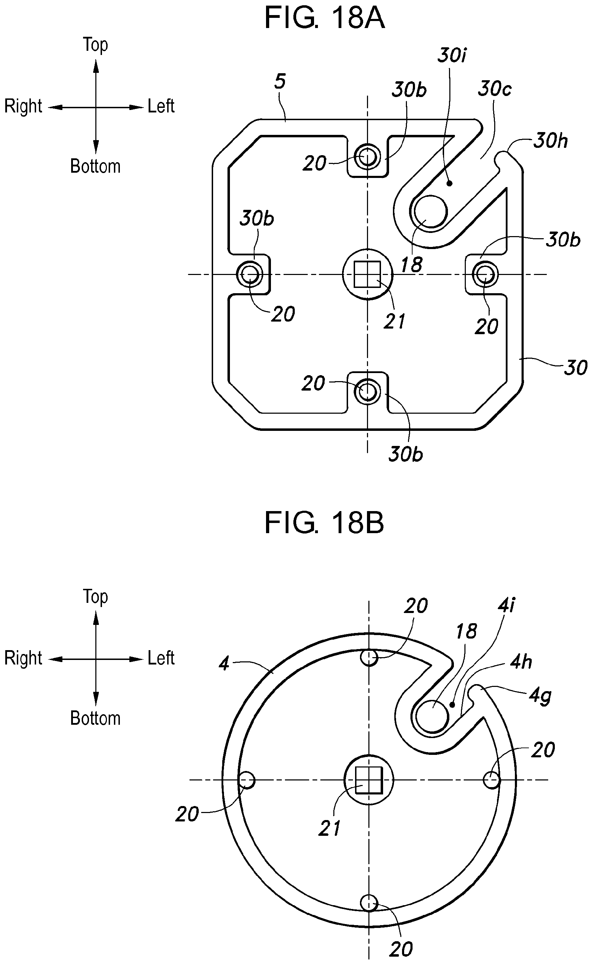

FIG. 17A is a view schematically illustrating a state in which rigid section 6 and transmission cable 18 are not mounted on insertion section 5, FIG. 17B is a view schematically illustrating a state in which rigid section 6 and transmission cable 18 are mounted on insertion section 5, FIG. 18A is a view schematically illustrating a cross section taken along line XVIIIa-XVIIIa of FIG. 17B, and FIG. 18B is a view schematically illustrating a cross section taken along line XVIIIb-XVIIIb of FIG. 17B.

Hereinafter, routing of transmission cable 18 according to the second exemplary embodiment will be described with reference to FIGs. 17A, 17B, 18A, and 18B. As illustrated in FIGs. 17A, 17B, and 18A, transmission cable 18 drawn out from rigid section 6 which is connected to support member 5g on the idle end side is stored in first groove portion 30c recessed from the outer surface in respective corner portions of joint piece 30. Transmission cable 18 is arranged on base end 5a from idle end 5b of insertion section 5 by sequentially passing through first groove portion 30c and is further stretched to grip section 2 through linear section 4.

As illustrated in FIG. 18A, wire conduction pieces 30b are provided in each side of each joint piece 30 vertically and horizontally, and control wires 20 are inserted into through holes formed in wire conduction pieces 30b. Spring joint 21 is inserted into the central portion of each joint piece 30 in the radial direction. In this manner, transmission cable 18 is arranged so as to be completely separated from a path on which control wire 20 and spring joint 21 are arranged and is accessible from the outside of insertion section 5.

Storage space 30i is formed in first groove portion 30c as a region whose cross-sectional area is larger than that of transmission cable 18 and the width of the opening of first groove portion 30c is formed to be smaller than the outer diameter of transmission cable 18. That is, tongue piece 30h projecting in the circumferential direction is longitudinally disposed in the opening of first groove portion 30c, and tongue piece 30h restricts the width of the opening in the circumferential direction to be small. In this manner, when insertion section 5 is bent, transmission cable 18 is movably formed in the inside of first groove portion 30c and separation of transmission cable 18 from first groove portion 30c is prevented. Tongue piece 30h is mainly deformed and transmission cable 18 is stored in storage space 30i by pushing transmission cable 18 against tongue piece 30h to be pushed in first groove portion 30c by the user or the like.

As illustrated in FIG. 18B, third groove portion 4h is disposed on the outer surface of linear section 4 in the longitudinal direction, and transmission cable 18 is stored in third groove portion 4h. Even in linear section 4, transmission cable 18 is completely separated from the path on which control wire 20 and spring joint 21 are arranged and is accessible from the outside of linear section 4. A groove is longitudinally provided on the outer surface of grip section 2 similar to linear section 4, and transmission cable 18 is stored therein. Storage space 4i whose cross-sectional area is larger than that of transmission cable 18 is formed in third groove portion 4h in the same manner as that of first groove portion 30c described above, tongue piece 4g is disposed in the longitudinal direction, and the width of the opening of third groove portion 4h is formed smaller than the outer diameter of transmission cable 18. In this manner, transmission cable 18 is movably formed in the inside of storage space 30i provided in linear section 5 and storage space 4i provided in linear section 4 in the longitudinal direction, and separation of transmission cable 18 from first groove portion 30c or third groove portion 4h in the outer diameter direction is prevented. Here, the sliding property of transmission cable 18 in storage spaces 30i and 4i is greatly improved and a load when insertion section 5 is bent can be reduced by means of using transmission cable 18 using polytetrafluoroethylene as a coating material.

In this manner, since transmission cable 18 drawn out from the rear end of rigid section 6 is stored along outer surfaces of insertion section 5, linear section 4, and grip section 2, a connector with a large size can be used as connector 18a which is provided in the terminal of transmission cable 18 and electrically connected with video processor 40 (see FIG. 1) and a connection operation becomes easy. Connector 18a becomes a disposable object (in the same manner as that of the third exemplary embodiment).

In the second exemplary embodiment, rigid section 6 is configured so as to be detachable from idle end 5b side of insertion section 5, and transmission cable 18 drawn out from rigid section 6 and the rear end of rigid section 6 is a disposable object. In this manner, since the number of disposable members is small, the cost required for the running, disposal, and recycling of endoscope 1 can be reduced to be low.

### [THIRD EXEMPLARY EMBODIMENT]

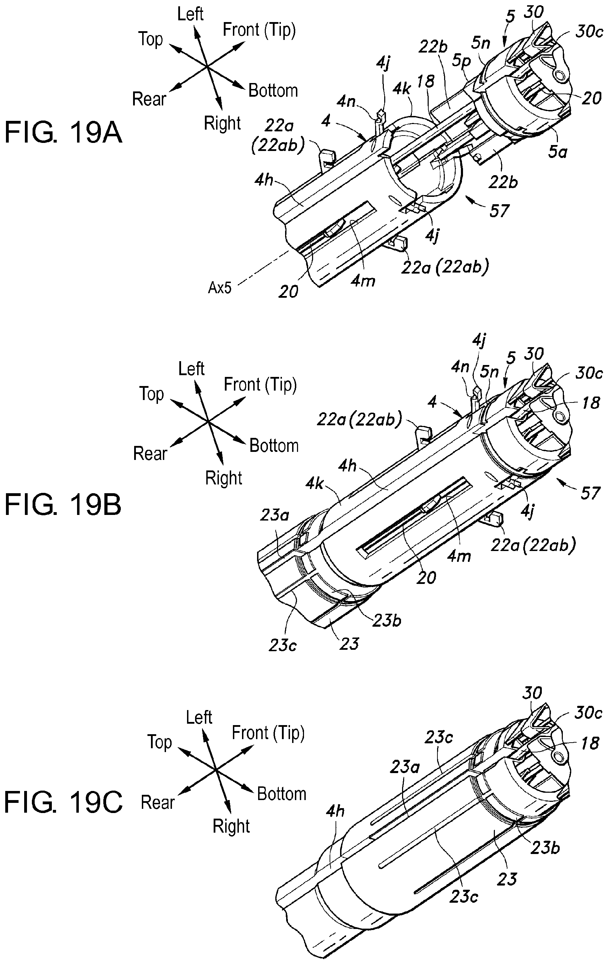

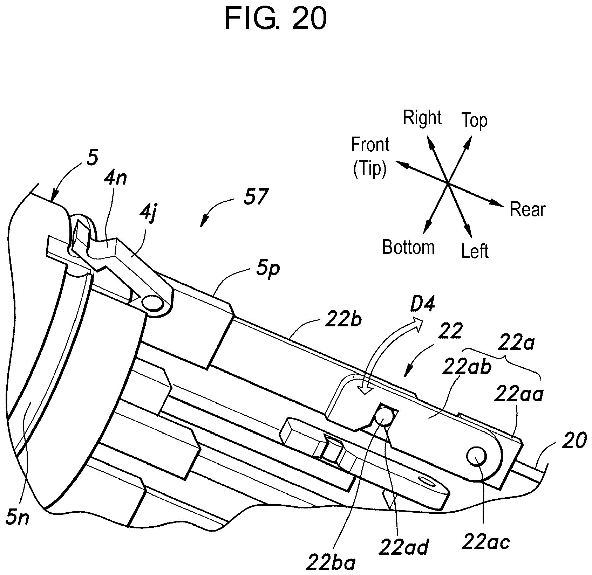

Figs. 19A, 19B, and 19C are perspective views illustrating a configuration of second connection section 57 which connects linear section 4 and insertion section 5 to each other in endoscope 1 according to a third exemplary embodiment of the present invention. FIG. 20 is a perspective view illustrating traction joint connection section 22 of control wire 20 in second connection section 57. The third exemplary embodiment adopts a configuration in which rigid section 6 is fixed to idle end 5b (refer to FIG. 2) of insertion section 5 so as not to be attachable and detachable, and proximal end 5a of insertion section 5 is attachable and detachable from a distal end of linear section 4. That is, rigid section 6, insertion section 5, and transmission cable 18 are disposable objects.

In order to realize this disposable aspect, a groove portion (third groove portion 4h) which supports transmission cable 18 so as to be attachable and detachable along the extending direction of linear section 4 disposed to extend on an outer surface of linear section 4 when insertion section 5 is mounted on linear section 4. Except for a configuration accompanied by newly provided second connection section 57, linear section 4, insertion section 5, and rigid section 6 have a configuration which is the same as that described in the first embodiment, and thus repeated description will be omitted herein.

Hereinafter, a configuration of second connection section 57 and a procedure for attaching and detaching insertion section 5 to and from linear section 4 will be described using FIGs. 19A, 19B, 19C, and 20. In FIG. 20, in order to facilitate description, cylindrical main body 4k of linear section 4 is illustrated transparently.

In the third exemplary embodiment, second connection section 57 includes a mechanism which is substantially th same as that of first connection section 56 (refer to FIG. 16) described above. That is, second movable locking claw 4j is disposed at three circumferential locations on an outer periphery near the distal end of linear section 4, and in addition, second fixing groove 5n is disposed in proximal end 5a of insertion section 5. Insertion section 5 is pressed against linear section 4 from the front, the distal end side of second movable locking claw 4j is moved pivotally and radially inward, and thus second movable locking claw 4j is stored in second fixing groove 5n.

In order to simplify the drawing, FIGs. 19A, 19B, and 19C omit the illustration of a mechanism which transmits rotation force to functional member displacement portion 6e of rigid section 6 (refer to FIGs. 7A, 7B, and 16). However, in practice, similar to first connection section 56 (refer to FIG. 16) described in the second exemplary embodiment, a shaft joint engaging portion (corresponding to shaft joint engaging portion 21a in FIG. 16) is disposed in the distal end of linear section 4 configuring second connection section 57, and a shaft joint engaged portion (corresponding to shaft joint engaged section 6f in FIG. 16) is disposed in the rear end of insertion section 5. If linear section 4 is fixed to insertion section 5, the shaft joint engaging portion and the shaft joint engaged portion are connected to each other, thereby transmitting the rotation force to rigid section 6.

Furthermore, in the third exemplary embodiment, insertion section 5 which is bendable is the disposable object. For this reason, as illustrated in FIGs. 19A and 20, second connection section 57 includes traction joint connection section 22 which transmits traction force generated on linear section 4 side to insertion section 5. Traction joint connection section 22 is configured to have four traction joint engaging portions 22a (however, FIG. 19A illustrates three of them) which are connected to the distal end of control wire 20 on the distal end side of linear section 4, and traction joint engaged portion 22b which is connected to the rear end of control wire 20 on proximal end 5a side of insertion section 5. That is, traction joint engaged portion 22b is disposed to correspond to traction joint engaging portions 22a.

Traction joint engaging portion 22a includes support piece 22aa and movable piece 22ab. Movable piece 22ab is pivotally supported by hinge portion 22ac disposed in support piece 22aa so that the distal end is pivotally movable in direction D4 (radial direction). Cutout portion 22ad is disposed on an outer surface facing radially inward in movable piece 22ab.

In contrast, traction joint engaged portion 22b is accommodated inside guide portion 5p disposed on an inner periphery of proximal end 5a of insertion section 5, and is adapted to be slidable in the longitudinal direction. In addition, projection 22ba projecting leftward in FIG. 20 is disposed in the rear of traction joint engaged portion 22b, and projection 22ba engages with cutout portion 22ad of movable piece 22ab described above, thereby allowing control wire 20 to be connected.

As illustrated in FIG. 19A, opening 4m which penetrates the front and rear surfaces of cylindrical main body 4k is formed along fifth axis Ax5 in linear section 4. Traction joint engaging portion 22a is arranged in a radially inner space from opening 4m. The same number (four, in this case) of opening 4m is disposed to correspond to the number of control wires 20 inserted into linear section 4 in the longitudinal direction. A user inserts forceps into opening 4m, grips and pulls movable piece 22ab radially outward. In this manner, movable piece 22ab is pivotally moved around hinge portion 22ac in direction D4 (radially outward) so that the distal end is exposed from opening 4m.

Hereinafter, description will be continued with reference to FIG. 19B. FIG. 19B illustrates a state where the rear end of insertion section 5 is temporarily mounted on linear section 4. In this state, second movable locking claw 4j protrudes outward from linear section 4, and movable piece 22ab of traction joint engaging portion 22a is exposed from opening 4m. Here, connection operation section 23 configured to have an elastic body such as a resin is disposed in the rear of linear section 4. Multiple slits 23c are formed rearward from the distal end in connection operation section 23. Fourth groove portion 23a which is orthogonal to the longitudinal direction and has a substantially U-shape in cross section is formed from the distal end to the rear end in an upper portion of connection operation section 23. Fourth groove portion 23a is guided to third groove portion 4h of linear section 4, thereby aligning connection operation section 23 and linear section 4 with each other in the circumferential direction. If the connection operation section 23 is moved forward, the width of slit 23c is broadened so that connection operation section 23 is slidable to linear section 4 in the longitudinal direction. Transmission cable 18 is arranged inside fourth groove portion 23a. A sectional shape of fourth groove portion 23a is not limited to the above-described substantial U-shape. As long as fourth groove portion 23a is guided to third groove portion 4h of linear section 4, any desired shape may be employed, and a bottomed shape such as the substantial U-shape may not be employed.

If connection operation section 23 is moved forward in a state where the rear end of insertion section 5 is temporarily mounted on linear section 4, the distal end of connection operation section 23 shortly comes into contact with movable piece 22ab of traction joint engaging portion 22a, thereby moving movable piece 22ab pivotally and radially inward. This causes cutout portion 22ad of movable piece 22ab to engage with projection 22ba (refer to FIG. 20) of traction joint engaged portion 22b, thereby connecting traction joint engaging portion 22a and traction joint engaged portion 22b to each other.

If the user moves connection operation section 23 further forward, the distal end of connection operation section 23 comes into contact with second movable locking claw 4j, second movable locking claw 4j is also moved pivotally and radially inward, and second movable locking claw 4j is accommodated in second fixing groove 5n.

The outer diameter of proximal end 5a of insertion section 5 is smaller than the outer diameter of the distal end of linear section 4. Second fixing groove 5n described above is formed to be deeper radially inward than proximal end 5a. In this manner, a step difference based on an outer diameter difference between the distal end of linear section 4 and second fixing groove 5n of insertion section 5 is formed in second connection section 57. In contrast, stepped portion 23b matching a shape of the step difference is disposed in the distal end of connection operation section 23. If the user moves connection operation section 23 further forward, as illustrated in FIGs. 19B to 19C, stepped portion 23b of connection operation section 23 is fitted to second fixing groove 5n and recess 4n of second movable locking claw 4j accommodated in second fixing groove 5n. This prevents connection operation section 23 from slipping out rearward. Stepped portion 23b presses second movable locking claw 4j accommodated in second fixing groove 5n radially inward, thereby fixing insertion section 5 to linear section 4 reliably.

If stepped portion 23b is fitted to second fixing groove 5n, connection operation section 23 entirely decreases in diameter, and the inner peripheral surface of connection operation section 23 comes into contact with the outer peripheral surface of linear section 4. In this manner, opening 4m is closed, and movable piece 22ab of traction joint engaging portion 22a exposed from opening 4m is pressed radially inward, thereby reliably fixing traction joint engaging portion 22a and traction joint engaged portion 22b to each other. That is, when insertion section 5 is mounted on linear section 4, connection operation section 23 maintains the connection between traction joint engaging portion 22a and traction joint engaged portion 22b. Then, the traction force serving to bend insertion section 5 is transmitted via traction joint engaging portion 22a and traction joint engaged portion 22b whose connection state is maintained by connection operation section 23.

Thereafter, the user presses the transmission cable 18 into fourth groove portion 23a of connection operation section 23 which overlaps third groove portion 4h of linear section 4. This causes transmission cable 18 to be arranged rearward. In FIG. 19A, in insertion section 5, transmission cable 18 is arranged on first groove portion 30c formed on the outer surface of joint piece 30 configuring insertion section 5. However, as will be described later, transmission cable 18 may be arranged inside insertion section 5.

With respect to used endoscope 1, the user detaches transmission cable 18 from third groove portion 4h and fourth groove portion 23a. Thereafter, the user moves connection operation section 23 rearward. In this manner, second movable locking claw 4j is released from second fixing groove 5n, and traction joint engaging portion 22a is released from traction joint engaged portion 22b. That is, when insertion section 5 is removed from linear section 4, connection operation section 23 disconnect traction joint engaging portion 22a and traction joint engaged portion 22b from each other. Using the forceps, the user can detach traction joint engaging portion 22a (movable piece 22ab) which is visible through opening 4m from traction joint engaged portion 22b (projection 22ba). Thereafter, if insertion section 5 is pulled forward, second movable locking claw 4j disengages from second fixing groove 5n, thereby enabling the user to easily pull out insertion section 5 from linear section 4.

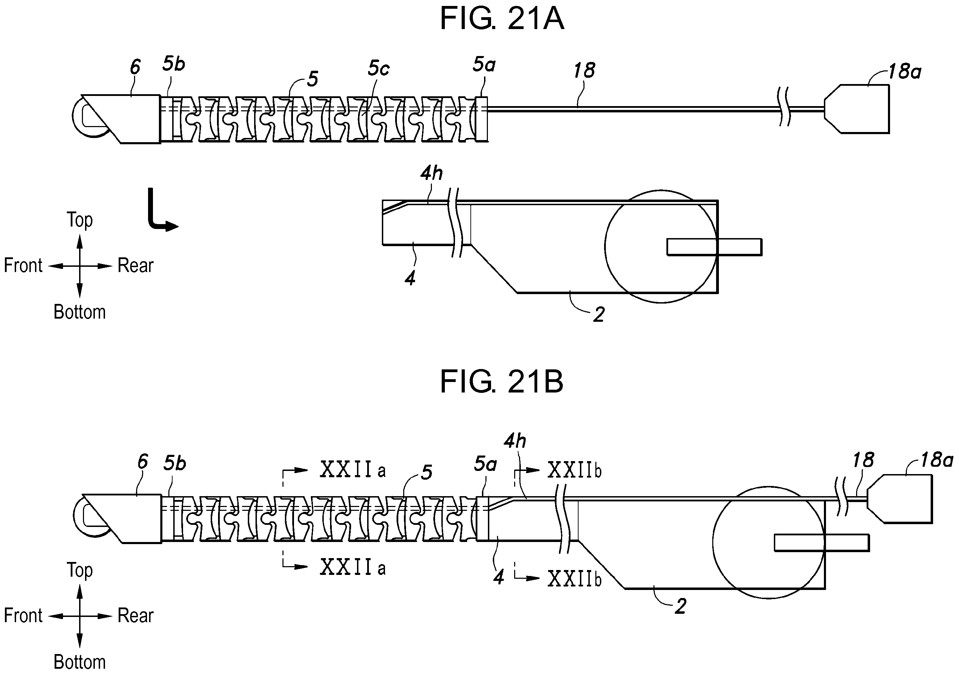

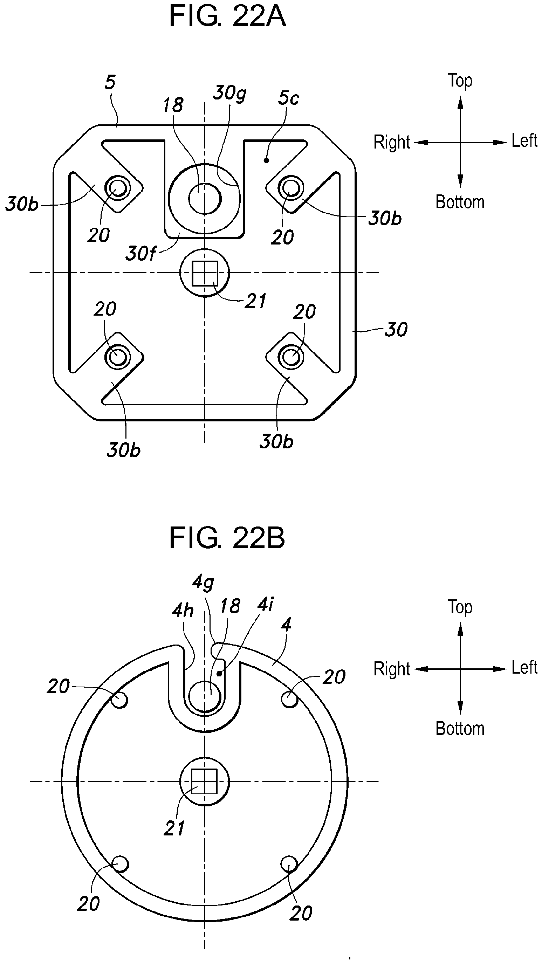

Fig. 21A is a schematic view illustrating a state where rigid section 6, insertion section 5 and transmission cable 18 have not yet been mounted on linear section 4. FIG. 21B is a schematic view illustrating a state where rigid section 6, insertion section 5, and transmission cable 18 are mounted on linear section 4. FIG. 22A is a schematic view illustrating a cross section taken line XXIIa-XXIIa in FIG. 21B. FIG. 22B is a schematic view illustrating a cross section taken line XXIIb-XXIIb in FIG. 21B. FIGs. 21A, 21B, and 22B omit the illustration of connection operation section 23 described above.

Hereinafter, arrangement of transmission cable 18 according to the third exemplary embodiment will be described with reference to FIGs. 21A, 21B, 22A, and 22B. Transmission cable 18 is arranged from the distal end of linear section 4 to the rear end of gripping section 2, that is, a section which is not the disposable object is arranged on the outer surface of endoscope 1. However, an arrangement position for insertion section 5 which is not the disposable object is not particularly limited.

As illustrated in FIG. 22A, in insertion section 5, transmission cable 18 may be inserted into through-hole 30g which penetrates cable guiding piece 30f projecting radially inward in a side portion of joint piece 30. In this case, transmission cable 18 is linearly pulled out rearward from the rear end of rigid section 6, and sequentially passes through through-hole 30g of each joint piece 30. As illustrated by a broken line in FIGs. 21A and 21B, transmission cable 18 extends inside hollow portion 5c formed along the extending direction of insertion section 5 from idle end 5b to proximal end 5a. This enables the user to easily attach and detach insertion section 5 to and from linear section 4 without being aware of transmission cable 18 arranged in insertion section 5. Transmission cable 18 is pulled out toward the outer surface of linear section 4 in proximal end 5a of insertion section 5.

Wire guiding piece 30b is disposed at each corner of each joint piece 30, and control wire 20 is inserted into a through-hole formed in wire guiding piece 30b. In addition, spring joint 21 is inserted into a radial center portion of each joint piece 30. Transmission cable 18 is arranged so as to be completely separated from routes for arranging control wire 20 and spring joint 21 by cable guiding piece 30f disposed inside insertion section 5 (joint piece 30).

As illustrated in FIGs. 21A, 21B, and 22B, third groove portion 4h extends in the longitudinal direction in the upper portion of linear section 4, and transmission cable 18 is accommodated in third groove portion 4h (or fourth groove portion 23a of connection operation section 23 which is overlapped with third groove portion 4h). Then, similar to linear section 4, a groove portion is also longitudinally disposed on the outer surface of gripping section 2, and transmission cable 18 extends thereto. Even in the third exemplary embodiment, accommodation space 4i which is larger than a cross-sectional area of transmission cable 18 may be formed in third groove portion 4h. Tongue piece 4g projecting in the circumferential direction may be disposed to extend longitudinally in an opening of third groove portion 4h, thereby causing tongue piece 4g to regulate an opening width in the circumferential direction to be small. In this manner, when insertion section 5 is bent, transmission cable 18 is movable inside third groove portion 4h, and transmission cable 18 is prevented from being separated from third groove portion 4h.

As described above, transmission cable 18 extends along linear section 4 and the outer surface of gripping section 2 which are not the disposable objects, and easily extend without being inserted into the inside thereof. Therefore, similar to the second exemplary embodiment, connector 18a of a large size can be used.

The third exemplary embodiment is configured so that insertion section 5 is attachable to and detachable from the distal end of linear section 4, and rigid section 6, insertion section 5, and transmission cable 18 are the disposable objects. As described above, even in the third exemplary embodiment, members which are the disposable objects are reduced to the minimum. Accordingly, it is possible to minimize the running cost of endoscope 1 and the cost required for discarding or recycling endoscope 1. In addition, since insertion section 5 and rigid section 6 are the disposable objects, it is no longer necessary to perform autoclave sterilization for these components, thereby saving the sanitary management cost of endoscope 1. Furthermore, since thermal resistance is not needed, insertion section 5 can be configured to have a resin instead of stainless steel, thereby saving the material cost.

Hitherto, the present invention has been described referring to the specific embodiments. However, these embodiments are merely examples. The present invention is not limited to these embodiments. For example, imaging unit 6a has been described as an example of the functional member in the embodiment. However, the functional member connected to idle end 5b of insertion section 5 may be a medical instrument such as a laser scalpel, an ultrasonic scalpel, forceps, and a snare used for polyp removal. In addition, each embodiment including a configuration where insertion section 5 is bendable has been described. However, the second exemplary embodiment and the third exemplary embodiment can also be applied to endoscope 1 including insertion section 5 having a fixed shape. That is, the present invention can also be applied to a configuration where rigid section 6 is directly arranged in the distal end of linear section 4.

All the respective configuration elements of the endoscope according to the present invention which are disclosed in the above-described embodiments are not necessarily essential, but can be appropriately and selectively employed within a range not departing from at least the scope of the present invention.

Endoscope 1 according to the present invention can rotate a captured image at any desired angle while maintaining an imaging direction in a state where insertion section 5 is bent. Therefore, the present invention can be preferably applied to endoscope 1 which images the inside of observation target which cannot be directly observed from the outside.

It is noted that the foregoing examples have been provided merely for the purpose of explanation and are in no way to be construed as limiting of the present invention. While the present invention has been described with reference to exemplary embodiments, it is understood that the words which have been used herein are words of description and illustration, rather than words of limitation. Changes may be made, within the purview of the appended claims, as presently stated and as amended, without departing from the scope and spirit of the present invention in its aspects. Although the present invention has been described herein with reference to particular structures, materials and embodiments, the present invention is not intended to be limited to the particulars disclosed herein; rather, the present invention extends to all functionally equivalent structures, methods and uses, such as are within the scope of the appended claims.

The present invention is not limited to the above described embodiments, and various variations and modifications may be possible without departing from the scope of the present invention. Further, features of the various alternate embodiments can be combined.

## Claims

1. An endoscope (1) comprising:
a grip section (2);
an insertion section (5) configured to be bendable, extend from a base end (5a) to an idle end (5b), and be rotatable about an extension direction thereof;
a functional member provided to the idle end (5b);
a plurality of control wires (20), trailing ends of which are fixed to the idle end (5b);
a traction member (8) provided to the base end (5a) and configured to tow starting ends of the plurality of control wires (20) so as to bend the insertion section (5);
a linear section (4) which is provided to the base end (5a) and is not bendable; and,
a spherical bearing (2c) which is provided at an opposite side of the linear section (4) with the traction member (8) interposed therebetween so that a coaxial degree of the spherical bearing (2c) with respect to the linear section (4) is maintained,
wherein, when the insertion section (5) is rotated, the functional member, the plurality of control wires (20), and the traction member (8) are rotated along with the insertion section (5), and the traction member (8) maintains a bending direction and bending angle of the insertion section (5) by changing a traction amount with respect to the plurality of control wires (20);
wherein the traction member (8) includes a rotation portion (8d) connected to the linear section (4),
the plurality of control wires (20) are respectively connected to different positions on an outer circumference of the rotation portion (8d), and
the rotation portion (8d) is inclinable with respect to a plane orthogonal to an axis of the linear section (4) and is rotated along with the linear section (4) in an inclined state; and,
wherein the traction member (8) includes a stationary portion (8c) that relatively rotatably supports the rotation portion (8d), and
the stationary portion (8c) is supported so as to be inclinable with respect to the plane orthogonal to the axis of the linear section (4) by the spherical bearing (2c), **characterized in that** the endoscope (1) further comprises a link member (10) arranged for transferring a force generated by a first operation portion (2a) provided on the grip section (2) of the endoscope (1) to the stationary portion (8c) for inclining the stationary portion (8c).

2. The endoscope (1) of claim 1, further comprising an inclination setting section configured to incline the stationary portion (8c) with respect to the plane orthogonal to the axis of the linear section (4) and to set an inclination direction and an inclination angle of the stationary portion (8c),
wherein the inclination setting section maintains the inclination direction and inclination angle of the stationary portion (8c) when viewed from a predetermined direction orthogonal to the axis of the linear section (4), when the rotation portion (8d) is rotated.

3. The endoscope (1) of claim 1, further comprising a rotation operation section provided on an outer circumference of the linear section (4) so as to axially rotate the linear section (4).

4. The endoscope (1) of claim 1, wherein the traction member (8) is configured of a bearing, the rotation portion (8d) includes an outer ring of the bearing, and the stationary portion (8c) includes an inner ring (81a) of the bearing.

5. The endoscope (1) of claim 1, wherein:
the linear section (4) is formed with a hollow portion extending in an axial direction,
the linear section (4) has a wire guide (9) that is provided on an outer circumference thereof and guides the plurality of control wires (20) to the hollow portion, and
the trailing ends of the plurality of control wires (20) guided by the wire guide (9) are fixed to an inner surface of the insertion section (5).

6. The endoscope (1) of claim 5, wherein each of the plurality of control wires (20) extends so as to reciprocate one or more times between the traction member (8) and the wire guide (9).

7. The endoscope (1) of claim 1, wherein the functional member is an imaging unit (6a) configured of an imaging device and an optical lens to focus incident light on the imaging device.

## Patentansprüche

1. Endoskop (1), mit:
einem Griffabschnitt (2),
einem Einführabschnitt (5), der ausgestaltet ist, biegsam zu sein, sich von einem Basisende (5a) zu einem freien Ende (5b) zu erstrecken und um seine Ausdehnungsrichtung rotierbar zu sein,
einem funktionalen Element, das an dem freien Ende (5b) vorgesehen ist,
mehreren Steuerdrähten (20), deren Rückenden an dem freien Ende (5b) fixiert sind,
einem Traktionselement (8), das an dem Basisende (5a) vorgesehen ist und ausgestaltet ist, an Vorderenden der mehreren Steuerdrähte (20) zu ziehen, um so den Einführabschnitt (5) zu biegen,
einem linearen Abschnitt (4), der an dem Basisende (5a) vorgesehen ist und der nicht biegsam ist, und
einem sphärischen Lager (2c), das an einer gegenüberliegenden Seite des linearen Abschnitts (4) vorgesehen ist, wobei das Traktionselement (8) dazwischen angeordnet ist, sodass ein koaxialer Grad des sphärischen Lagers (2c) hinsichtlich des linearen Abschnitts (4) beibehalten wird,
wobei, wenn der Einführabschnitt (5) rotiert wird, das funktionale Element, die mehreren Steuerdrähte (20) und das Traktionselement (8) zusammen mit dem Einführabschnitt (5) rotiert werden, und das Traktionselement (8) eine Biegungsrichtung und einen Biegungswinkel des Einführabschnitts (5) durch Ändern eines Traktionsausmaßes hinsichtlich der mehreren Steuerdrähte (20) beibehält,
wobei das Traktionselement (8) einen Rotationsabschnitt (8d) aufweist, der mit dem linearen Abschnitt (4) verbunden ist,
die mehreren Steuerdrähte (20) jeweils mit unterschiedlichen Positionen auf einem äußeren Umfang des Rotationsabschnitts (8d) verbunden sind und
der Rotationsabschnitt (8d) hinsichtlich einer Ebene orthogonal zu einer Achse des linearen Abschnitts (4) neigbar ist und mit dem linearen Abschnitt (4) in einem geneigten Zustand rotiert wird und
wobei das Traktionselement (8) einen stationären Abschnitt (8c) aufweist, der den Rotationsabschnitt (8d) relativ drehbar stützt, und
der stationäre Abschnitt (8c) durch das sphärische Lager (2c) derart gestützt wird, dass er hinsichtlich der Ebene senkrecht zu der Achse des linearen Abschnitts (4b) neigbar ist,
**gekennzeichnet dadurch, dass**
das Endoskop (1) ferner ein Verbindungselement (10) aufweist, das ausgestaltet ist, eine Kraft, die durch einen ersten Bedienungsabschnitt (2a), der an dem Griffabschnitt (2) des Endoskops (1) vorgesehen ist, erzeugt wird, an den stationären Abschnitt (8c) zum Neigen des stationären Abschnitts (8c) zu übertragen.

2. Endoskop (1) nach Anspruch 1, ferner mit einem Neigungseinstellabschnitt, der ausgestaltet ist, den stationären Abschnitt (8c) hinsichtlich der Ebene orthogonal zu der Achse des linearen Abschnitts (4) zu neigen und eine Neigungsrichtung und einen Neigungswinkel des stationären Abschnitts (8c) einzustellen,
wobei der Neigungseinstellabschnitt die Neigungsrichtung und den Neigungswinkel des stationären Abschnitts (8c) aus Sicht einer vorbestimmten Richtung orthogonal zu der Achse des linearen Abschnitts (4) beibehält, wenn der Rotationsabschnitt (8d) rotiert wird.

3. Endoskop (1) nach Anspruch 1, ferner mit einem Rotationsbedienungsabschnitt, der an einem äußeren Umfang des linearen Abschnitts (4) vorgesehen ist, um axial den linearen Abschnitt (4) zu rotieren.

4. Endoskop (1) nach Anspruch 1, wobei das Traktionselement (8) aus einem Lager gebildet ist, wobei der Rotationsabschnitt (8d) einen äußeren Ring des Lagers umfasst und der stationäre Abschnitt (8c) eine inneren Ring (81 a) des Lagers umfasst.

5. Endoskop (1) nach Anspruch 1, wobei:
der lineare Abschnitt (4) mit einem hohlen Abschnitt gebildet ist, der sich in einer axialen Richtung erstreckt,
der lineare Abschnitt (4) eine Drahtführung (9) aufweist, die an einem äußeren Umfang davon vorgesehen ist und die mehreren Steuerdrähte (20) zu dem hohlen Abschnitt führt, und
die Rückenden der mehreren Steuerdrähte (20), die durch die Drahtführung (9) geführt sind, an einer inneren Oberfläche des Einführabschnitts (5) fixiert sind.

6. Endoskop (1) nach Anspruch 5, wobei jeder der mehreren Steuerdrähte (20) sich so erstreckt, dass er einmal oder mehrmals zwischen dem Traktionselement (8) und der Drahtführung (9) hin- und herverläuft.

7. Endoskop (1) nach Anspruch 1, wobei das funktionale Element eine Bildaufnahmeeinheit (6a) ist, die aus einer Bildaufnahmevorrichtung und einem optischen Objektiv zum Fokussieren von einfallendem Licht auf der Bildaufnahmevorrichtung gebildet ist.

## Revendications

1. Endoscope (1) comprenant:
une section de préhension (2),
une section d'insertion (5) qui est configurée pour être pliable, pour s'étendre d'une extrémité de base (5a) à une extrémité libre (5b) et pour pouvoir tourner autour de sa direction d'extension,
un élément fonctionnel qui est prévu à l'extrémité libre (5b),
une pluralité de fils de commande (20) dont les extrémités arrière sont fixées à l'extrémité libre (5b),
un élément de traction (8) qui est prévu à l'extrémité de base (5a) et configuré pour tirer sur des extrémités avant de la pluralité de fils de commande (20) de manière à plier la section d'insertion (5),
une section linéaire (4) qui est prévue à l'extrémité de base (5a) et qui n'est pas pliable, et
un palier sphérique (2c) qui est prévu sur un côté opposé de la section linéaire (4), avec l'élément de traction (8) intercalé entre eux, de telle sorte qu'un degré coaxial du palier sphérique (2c) par rapport à la section linéaire (4) est maintenu,
dans lequel, lorsque la section d'insertion (5) est tournée, ledit élément fonctionnel, ladite pluralité de fils de commande (20) et ledit élément de traction (8) sont tournés conjointement avec la section d'insertion (5), et l'élément de traction (8) maintient une direction de pliage et un angle de pliage de la section d'insertion (5) en changeant une amplitude de traction par rapport à la pluralité de fils de commande (20),
dans lequel l'élément de traction (8) comprend une portion de rotation (8d) qui est reliée à la section linéaire (4),
lesdits plusieurs fils de commande (20) sont reliés chacun à des positions différentes sur une circonférence extérieure de la portion de rotation (8d), et
la portion de rotation (8d) peut être inclinée par rapport à un plan orthogonal à un axe de la section linéaire (4) et est tournée conjointement avec ladite section linéaire (4) dans un état incliné, et
dans lequel ledit élément de traction (8) comprend une portion stationnaire (8c) qui supporte à rotation relative la portion de rotation (8d), et
ladite portion stationnaire (8c) est supportée par le palier sphérique (2c) de manière à être inclinable par rapport au plan orthogonal à l'axe de la portion linéaire (4),
**caractérisé par le fait que**
l'endoscope (1) comprend en outre un élément de jonction (10) qui est agencé pour transférer une force générée par une première portion de manoeuvre (2a) prévue sur ladite section de préhension (2) de l'endoscope (1), à la portion stationnaire (8c) pour incliner la portion stationnaire (8c).

2. Endoscope (1) selon la revendication 1, comprenant en outre une section de réglage d'inclinaison configurée pour incliner la portion stationnaire (8c) par rapport au plan orthogonal à l'axe de la section linéaire (4) et pour régler une direction d'inclinaison et un angle d'inclinaison de la portion stationnaire (8c),
dans lequel la section de réglage d'inclinaison maintient la direction d'inclinaison et l'angle d'inclinaison de la portion stationnaire (8c), vu à partir d'une direction prédéterminée orthogonale à l'axe de la section linéaire (4), lorsque la portion de rotation (8d) est tournée.

3. Endoscope (1) selon la revendication 1, comprenant en outre une section de manoeuvre de rotation qui est prévue sur une circonférence extérieure de la section linéaire (4) de manière à tourner axialement la section linéaire (4).

4. Endoscope (1) selon la revendication 1, dans lequel ledit élément de traction (8) est constitué d'un roulement, dans lequel ladite portion de rotation (8d) comprend une bague extérieure du roulement et la portion stationnaire (8c) comprend une bague intérieure (81a) du roulement.

5. Endoscope (1) selon la revendication 1, dans lequel:
ladite section linéaire (4) est formée avec une portion creuse s'étendant dans une direction axiale,
la section linéaire (4) présente un guide-fil (9) qui est prévu sur une circonférence extérieure de celle-ci et qui guide la pluralité de fils de commande (20) vers la portion creuse, et
les extrémités arrière de la pluralité de fils de commande (20) qui sont guidés par le guide-fil (9) sont fixées sur une surface intérieure de la section d'insertion (5).

6. Endoscope (1) selon la revendication 5, dans lequel chacun de la pluralité de fils de commande (20) s'étend de manière à aller et venir une ou plusieurs fois entre l'élément de traction (8) et le guide-fil (9).

7. Endoscope (1) selon la revendication 1, dans lequel l'élément fonctionnel est une unité de prise d'image (6a) qui est constituée d'un dispositif de prise d'image et d'un objectif optique destiné à focaliser de la lumière incidente sur le dispositif de prise d'image.
